(19) Europäisches Patentamt
European Patent Office
Office européen des brevets

(11) **EP 4 050 609 B1**

(12) **EUROPEAN PATENT SPECIFICATION**

(45) Date of publication and mention
of the grant of the patent:
**29.10.2025 Bulletin 2025/44**

(21) Application number: **21212932.4**

(22) Date of filing: **24.04.2013**

(51) International Patent Classification (IPC):
**G16B 15/20** (2019.01)    **G16B 40/00** (2019.01)
**G16B 40/20** (2019.01)    **G16B 15/30** (2019.01)

(52) Cooperative Patent Classification (CPC):
**G16B 15/20; G16B 40/20;** G16B 15/30; G16B 40/00

(54) **METHODS AND SYSTEMS FOR IDENTIFICATION OF A PROTEIN BINDING SITE**

VERFAHREN UND SYSTEME ZUR IDENTIFIKATION EINER PROTEINBINDUNGSSTELLE

PROCÉDÉS ET SYSTÈMES D'IDENTIFICATION D'UN SITE DE LIAISON PROTÉIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **24.04.2012   US 201261637849 P
08.09.2012   US 201261698614 P
10.01.2013   US 201361751124 P
15.03.2013   US 201361788529 P**

(43) Date of publication of application:
**31.08.2022   Bulletin 2022/35**

(62) Document number(s) of the earlier application(s) in
accordance with Art. 76 EPC:
**13782203.7 / 2 842 068**

(73) Proprietor: **Laboratory Corporation of America
Holdings
Burlington, North Carolina 27215 (US)**

(72) Inventors:
• **EVANS, Mark
Pleasant Hill, CA 94523 (US)**
• **HADDAD, Mojgan
Orinda, CA 94563 (US)**

(74) Representative: **Forresters IP LLP
Skygarden
Erika-Mann-Straße 11
80636 München (DE)**

(56) References cited:
WO-A1-2011/119484     WO-A2-01/36980
WO-A2-02/44990        US-A1- 2003 013 113

• **HENRICH S. ET AL: "Computational approaches
to identifying and characterizing protein binding
sites for ligand design", JOURNAL OF
MOLECULAR RECOGNITION, 1 January 2009
(2009-01-01), pages n/a - n/a, XP055052028,
ISSN: 0952-3499, DOI: 10.1002/jmr.984**

**Description**

PRIOR RELATED APPLICATIONS

This application claims priority to U.S. provisional application No. 61/637,849 filed April 24, 2012, U.S. provisional application No. 61/698,614 filed September 8, 2012, U.S. provisional application No. 61/751,124 filed January 10, 2013, and U.S. provisional application No. 61/788,529 filed March 15, 2013.

FIELD

The present invention is directed to computer-related methods and systems for identification of ligand binding sites of a protein, such as epitopes of antibodies on protein antigens.

BACKGROUND

Investigation of binding sites on protein molecules is an important area of inquiry in the field of biomedical research. The examination of binding sites is important for the functional characterization of protein molecules, the understanding of interactions of the proteins with their ligands, the design of protein binding sites and their ligands, and other purposes. Binding of proteins to other proteins, generally referred to as protein-protein interactions, or other biological molecules, underpins a variety of biochemical processes at molecular, cellular, and physiological levels. One illustration is the functioning of the adaptive immune system in jawed vertebrates. A crucial feature of the adaptive immune response is the production of antibody molecules that bind antigens with high specificity. Highly specific binding of the antibodies to antigens allows for a tailored physiological response, the goal of which is neutralization and destruction of "non-self" molecules, cells, or organisms, such as viruses, bacteria, parasites, and other pathogens, or cancer cells.

The effectiveness of vaccines, which are immunogenic compositions administered in order to confer on their recipients the ability to mount effective immune protection upon exposure to a "non-self" molecule, cell, or organism relies on induction of an effective adaptive immune response, including production of effective neutralizing antibodies. Development of an effective vaccine can be greatly aided by understanding the mechanism of action of neutralizing antibodies and by the identification of epitopes that can induce effective immune responses in a vaccine's recipient. For example, significant efforts in human vaccine design are currently focused on immunogens that evoke neutralizing antibody responses to a broad spectrum of human immunodeficiency viruses (HIV) circulating worldwide. Developing an understanding of the binding of broadly neutralizing antibodies to HIV virus is useful for the development of an effective HIV vaccine.

What is needed, therefore, are methods and systems for the accurate and efficient identification of binding sites of protein molecules (*e.g.*, sites that interact with a neutralizing antibody, that interact with another protein, or that interact with a small molecule). Methods and systems for the production of immunogenic compositions also are needed.

WO2011/119484 discloses methods of identifying peptide binding to ligands. In particular. this document describes methods for identification in silico of peptides and sets of peptides internal to or on the surface of microorganisms and cells which have a high probability of being effective in stimulating humoral and cell mediated immune response.

SUMMARY

The invention is set out in the appended set of claims. The terms and phrases "invention," "the invention," "this invention," and "the present invention" are intended to refer broadly to all of the subject matter described herein or claimed. Statements containing one of these terms or phrases should be understood not to limit the subject matter described herein or to limit the meaning or scope of the claims. The claimed embodiments of the invention are defined by the claims, not this summary. This summary is a high-level overview of various aspects of the invention and introduces some of the concepts that are further described in the Detailed Description section below. This summary is not intended to identify key or essential features of the claimed subject matter, nor is it intended to be used in isolation to determine the scope of the claimed subject matter. The subject matter should be understood by reference to appropriate portions of the entire specification, any or all drawings, and each claim.

The computer-implemented methods of the present invention provide processes for identification of ligand binding sites on protein molecules. One example of such a binding site is a binding site for an antibody on a protein antigen molecule, also commonly referred to as an "epitope." However, the methods of the present invention are broadly applicable to identification of various types of binding sites on proteins, including protein-protein binding sites and protein-small molecule binding sites. In some embodiments, the binding site may include "binding pockets," or crevices within proteins that small molecules may bind. This is somewhat opposite of antibody binding, which tends to favor features that project from the protein surface. Either site can be mapped using the described patch approach. The computer-implemented methods of the present invention use a combination of protein sequence data, structural information, experimental data on binding affinity, and computational modeling in order to identify ligand binding sites on protein molecules. In one example, the methods of the present invention are used for identification of epitopes for antibodies on a protein molecule. The computer-implemented methods comprise obtaining sequence information for a plurality of sequence variants of the protein; obtaining the measure of the biological activity of each of the plurality of the sequence variants for the ligand; using a three-dimensional model of the protein's structure to define patches on the protein structure containing a plurality of residues within close proximity of each other, wherein the residue at the center of the patch is determined based on solvent accessibility, and wherein the residues are within 20 angstroms of the residue at the center; correlating the sequence variability within each of the patches with the biological activity data for each sequence variant, wherein the information on the sequence variability within each area and the data on affinity for each sequence variant is represented in a non-binary or binary form, compiled as a vector file, and processed by two or more computational methods testing each patch for an ability of the sequence variability within the area to predict changes in the biological activity; wherein the two or more computational methods comprise i) at least one method involving multivariate analysis, at least one method involving univariate analysis, and at least one machine learning algorithm, or ii) two or more of multiple regression analysis, logistic regression analysis, support vector machine, Fischer's Exact test, Hidden Markov Models, Neural Networks, Random Forest, Decision Trees, or Bayesian Networks; and wherein a residue is identified as a potential binding site residue if variation at the residue exhibits statistically significant correlation with the biological activity; identifying the residues forming the binding site by comparing the potential binding site residues identified with the two or more computational methods and identifying residues based on the agreement between the two or more computational methods; and producing a vaccine or other immunogenic composition comprising an immunogen and a pharmaceutically acceptable carrier, wherein the immunogen comprises the binding site identified above; or producing an antibody that interacts with the binding site identified above, wherein the antibody is effective to disrupt the interaction of the protein with the ligand. In some embodiments of the methods described herein, the information on the binding sites obtained for variations of a ligand or for different ligands is analyzed for high-level identification of the areas of a protein that are involved in binding of a particular type of ligand or ligand binding generally. In certain embodiments, the data on affinity is obtained by conducting suitable experiments for measuring affinity between the protein and its ligand, for example, but not limited to, by determining the dissociation constant, by determining the Michaelis constant, or by determining the $IC_{50}$ of a particular biological response of the protein or its ligand. In some embodiments, the methods are used to identify a binding site for a neutralizing antibody. In some embodiments, the three-dimensional model is from a crystal structure or computer-generated model. In some embodiments of the methods, the two or more computational methods comprise at least one method involving multivariate analysis, at least one method involving univariate analysis, and at least one machine learning algorithm. In certain embodiments, the two or more computational methods comprise multiple regression analysis, logistic regression analysis, support vector machine, Fischer's Exact test, Hidden Markov Models, Neural Networks, Random Forest, Decision Trees, or Bayesian Networks, or a combination thereof. The methods may comprise, for example, two computational methods, three computational methods, or four or more computational methods. In certain embodiments, the computational methods comprise using multiple linear regression, support vector machine, and Fischer's Exact test analyses.

Administering to an individual a vaccine or other immunogenic composition based on the binding site identified. In some embodiments, administering the immunogenic composition to an individual confers upon the individual the ability to mount an effective immune response upon exposure to the protein, or cell or organism that comprises the protein.

In other embodiments, the methods are used to identify a binding site for the protein's interaction with another protein or small molecule. The methods further include the step of producing an antibody based on the binding site identified by the present methods and/or optionally administering to an individual a composition comprising the antibody and a pharmaceutically acceptable carrier. In some aspects that do not form part of the invention, the antibody is administered in an amount effective to disrupt the interaction of the protein with the other protein or small molecule.

In other aspects that do not form part of the invention, vaccines or other immunogenic compositions are provided comprising at least one binding site or a portion thereof and a pharmaceutically acceptable carrier, wherein the binding site or portion thereof was identified by the present methods. In certain aspects, the vaccines or other immunogenic compositions are effective to confer upon an individual the ability to mount an effective immune response upon exposure to the protein, or cell or organism that comprises the protein. In some aspects, the vaccine or other immunogenic composition includes two, three, or more protein binding sites or portions thereof.

In other aspects that do not form part of the invention, antibodies are provided that interact with a protein binding site or portion thereof identified by the present methods. In certain aspects, the antibodies are present in a pharmaceutical composition with a pharmaceutically acceptable carrier. In certain aspects, the antibodies are effective to disrupt the interaction of the protein with another protein or small molecule.

In other aspects that do not form part of the invention, methods for inducing an immune response in an individual are provided. These methods may include identifying a ligand binding site of a protein using the binding site identification methods disclosed in this application; producing a vaccine or other immunogenic composition comprising an immunogen and a pharmaceutically acceptable carrier, wherein the immunogen comprises the binding site identified in the present methods; and administering to the individual the vaccine or other immunogenic composition in an amount effective to induce an immune response in the individual upon exposure to the protein or a cell or organism that comprises the protein.

In other aspects that do not form part of the invention, methods for disrupting the interaction of a protein and its ligand in an individual are provided. These methods include in some aspects identifying a ligand binding site of a protein using the binding site identification methods disclosed in this application; producing an antibody that interacts with the binding site identified in the present methods; and administering to the individual a composition comprising the antibody and a pharmaceutically acceptable carrier, wherein the antibody is in an amount effective to disrupt the interaction of the protein and its ligand.

Also provided are systems for implementing the computer-implemented method of the invention. These systems include, in certain embodiments, a computer readable medium; and a processor in communication with the computer readable medium, the program configured to receive sequence information for a plurality of sequence variants of the protein; receive affinity data or a viable surrogate of affinity data of each of the plurality of the sequence variants for the ligand; receive data regarding a three-dimensional model of the protein's structure; apply two or more computational methods to the data; compare the results obtained by applying the computational methods; and identify at least one residue forming the binding site. In some embodiments, the system further includes at least one database in communication with the processor, wherein the at least one database comprises the sequence data, the affinity data, the three-dimensional model data, or a combination thereof. The two or more computational methods include at least one method involving multivariate analysis, at least one method involving univariate analysis, and at least one machine learning algorithm. The two or more computational methods comprise multiple regression analysis, logistic regression analysis, support vector machine, Fischer's Exact test, Hidden Markov Models, Neural Networks, Random Forest, Decision Trees, or Bayesian Networks, or a combination thereof.

In another aspect, computer readable media are provided. The computer readable media may include program code including program code for receiving sequence data, the sequence data representing an amino acid sequence, nucleic acid sequence, or both of at least a portion of protein; program code for receiving affinity data; program code for receiving data regarding a three-dimensional model of the protein's structure; program code for applying two or more computational methods to the data; program code for comparing the results obtained by applying the computational methods; and program code for identifying at least one residue forming the binding site.

## BRIEF DESCRIPTION OF THE FIGURES

Figure 1A is a schematic representation of one embodiment of a method of protein binding site identification.

Figure 1B schematically illustrates the flow of information in an exemplary system for carrying out the methods of identification of a protein binding site.

Figure 2 is a schematic representation of the Residue Interaction Network (RIN) concept. In this embodiment, the first layer (Level 1) is at depth 0 angstroms, the second layer (Level 2) at 1.5 angstroms, and the third layer (Level 3) at 2.5 angstroms. These depths (*e.g.*, 1.5, 2.5) are experimentally determined distribution median depths. Actual depths of amino acid residues run on a continuum, for example, of 0-6 or even 9 angstroms and may go deeper in some embodiments.

Figure 3 is a schematic representation of an example of a surface patch defined at different mean residue atomic depths (0 angstroms, 1.5 angstroms, and 2.5 angstroms).

Figure 4 is a schematic representation of an example of a surface patch defined at different mean residue atomic depths (2.5 angstroms 0 angstroms) in a space filling model.

Figure 5 is a schematic representation of a method of protein binding site identification, as applied to epitope prediction process.

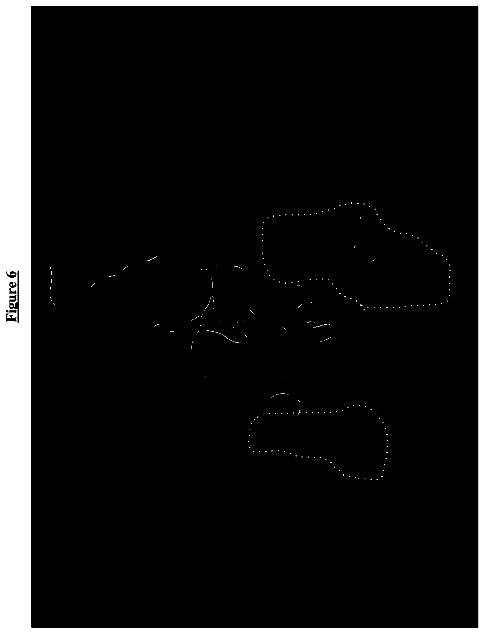

Figure 6 is a schematic representation of the existing HIV-1 gp120 models used in the gp120 epitope prediction process. The CPH model is shown in blue (or light gray). The 3JWD model is shown in red or (dark gray). The dotted line indicates the areas of the predicted protein model which do not correspond to the 3JWD crystal structure and depicts their shape. These regions represent loops, which are truncated in most crystal models because of their high degree of vibration. Loop regions are often important in protein interactions; however, it is understood that predicted loop structures may be very inaccurate. Therefore, the loop sequences are included in this representation to illustrate that they are included when patches are defined in embodiments of the present methods. Software specifically designed for loop structure optimization may be used in certain embodiments, resulting in multiple patch sets which could then be evaluated through the present methods.

Figure 7 are graphs representing an example of experimental results of $IC_{50}$ determination.

Figures 8A and 8B provide an example of a patch file. Figure 8B is a continuation of Figure 8A.

Figure 9 is a schematic representation illustrating a process of patch alignment. Generally, the structures with green/blue (or light and dark gray) color-coding are showing epitopes that occur very frequently in significant patches. The potential epitope residues appearing with higher frequency in the analyzed patches are colored green (dark gray). The potential epitope residues appearing with the lower level of frequency are colored blue (light gray, around the dark gray region).

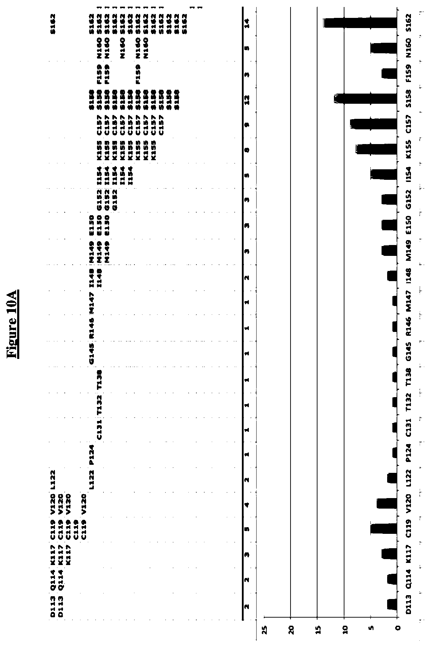

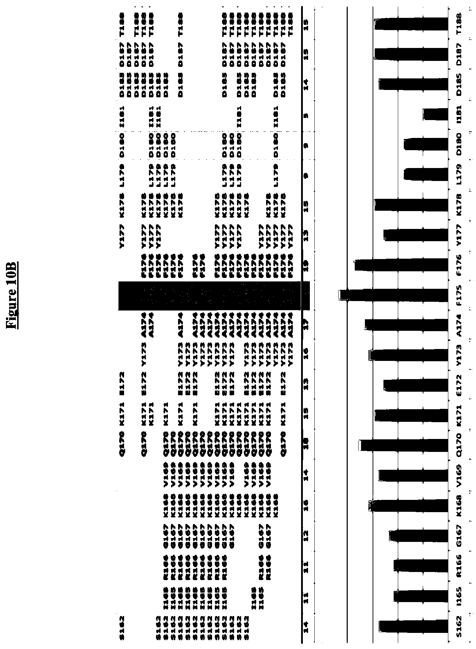

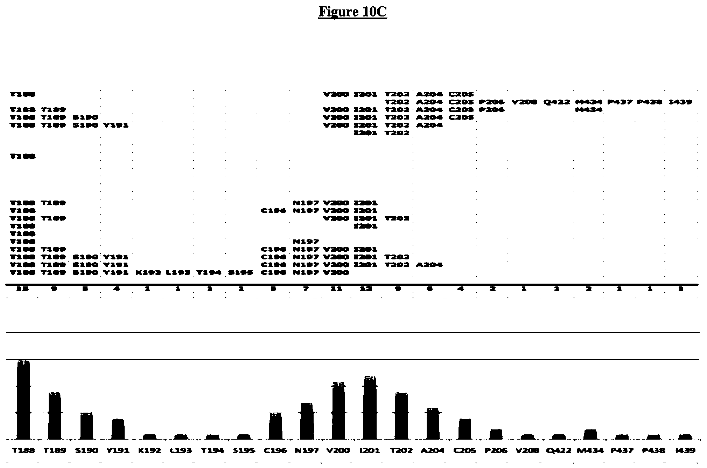

Figures 10A-10C provide a schematic representation of patch alignment used for identification of an epitope for the PG9 antibody on the gp120 protein. Figures 10A, 10B, and 10C are parts of the same figure, with some overlap at the edges for easier alignment of the images. These figures show the spatial organization of the residues comprising a patch in linear space. In the upper panels, patch residues that arise from the same position number are vertically aligned. After many patches are thus aligned, the frequency at which any particular residue position occurs in the analyzed patches can be determined. The exemplary display is color-coded analogously to a heat map, with the most frequently occurring residues shown in red, less frequently occurring residues shown in orange, even less frequently occurring residues shown in yellow, and the least frequently occurring residues shown in green (all shown as shaded in gray). Residues that fall within color-coded or shaded regions are considered to be high probability interaction residues and are therefore termed "primary epitope residues." Remaining residues in the figures with a frequency of occurrence lower than that for the residues occurring in green are considered lower probability interaction residues and are termed "secondary epitope residues." The bar graph in the lower panels represents the same information. The y-axis shows the frequency of the residue, and the x-axis shows the position of the residue.

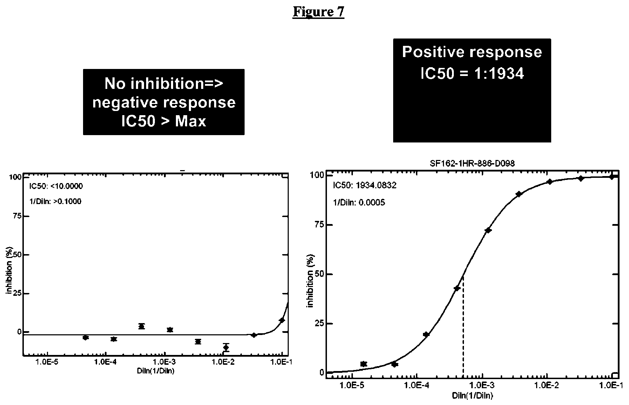

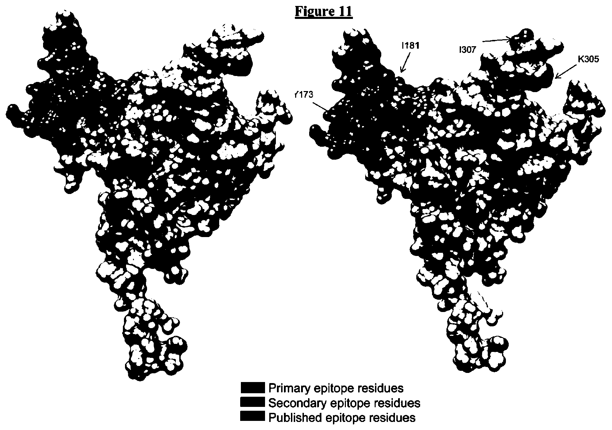

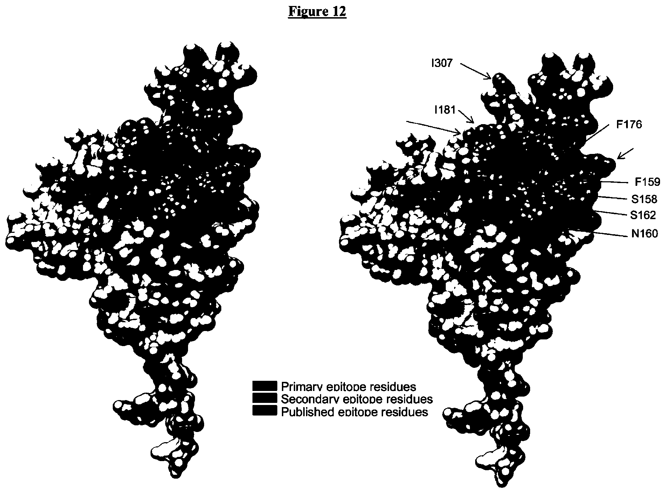

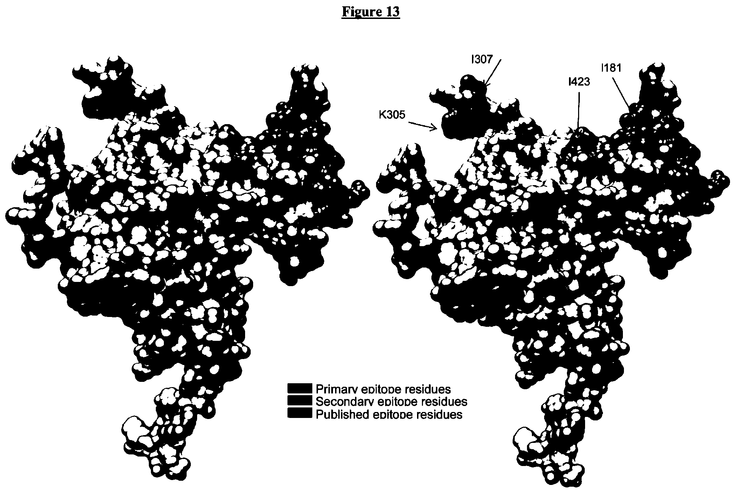

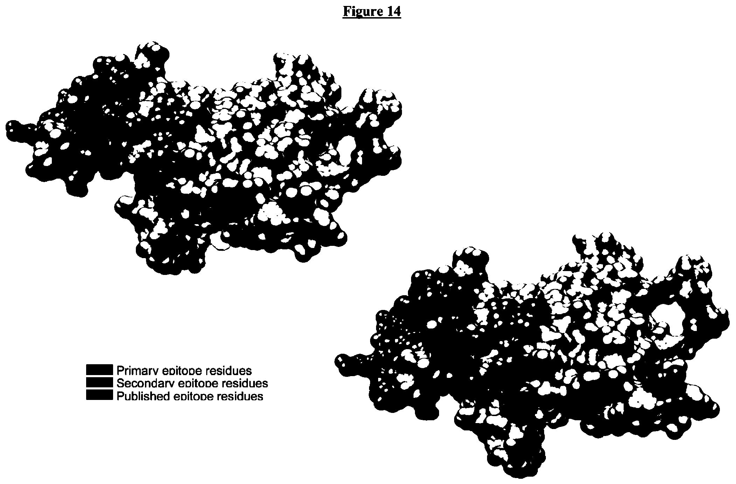

Figures 11-14 are schematic representations of different views of the gp120 protein structure with predicted and published PG9 epitopes identified. In each of these figures, primary epitope residues are shown in green (dark gray); secondary epitope residues are shown in blue (light gray); and previously published epitope residues are shown in red (light gray) and indicated by the arrows and the indication of the residue and position. In these exemplary embodiments, the primary and secondary epitope residues were determined as discussed above for Figure 7. The previously published epitope residues are described, for example, in Walker et al. (2009, "Broad and potent neutralizing antibodies from an African donor reveal a new HIV-1 vaccine target." Science 326(5950):285-89). A "stereo" view of two models is shown, with the left view showing epitopes identified using the present methods, while the right view shows where the newly identified and published epitopes overlap.

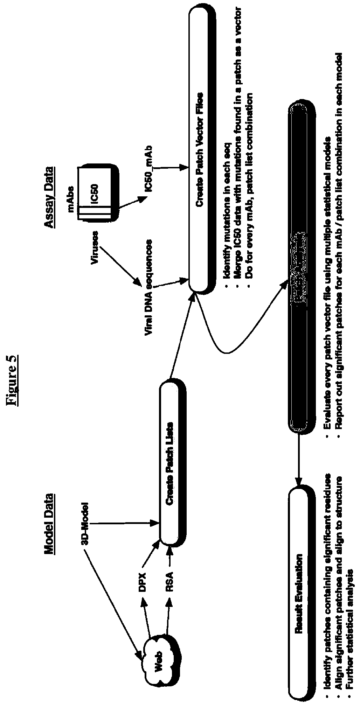

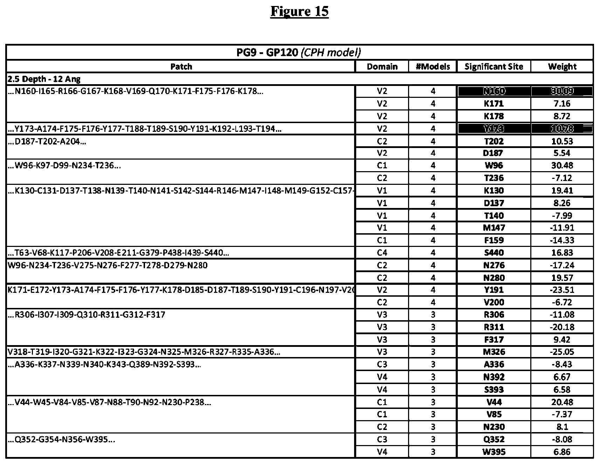

Figure 15 is a table summarizing selected information on the patches used for PG9 epitope identification on gp120 protein using the CPH model. In this table, data from one entry of the 3x3 matrix in Figure 5 is shown (*i.e.*, a patch that is 12 angstroms in diameter and 2.5 angstroms deep). The domain column refers to commonly known functional domains within gp120. The column "#Models" refers to how many of the 4 statistical methods (Multiple Linear Regression, Support Vector Machine, Logistic Regression, Fisher exact test) called a particular patch residue as significant. The "significant site" column identifies which residue in the patch was called significant. The weight column refers to the odds ratio for a residue, which identifies how significant it was.

Figure 16 is a table summarizing information on 30 patch sites used for PG9 epitope identification. The columns in this table are the same as those in Figure 15, sorted by position.

Figure 17 is a table summarizing the sequence differences between two HIV-1 clones with distinct $IC_{50}$ values for PG9 antibody. The 0 or 1 refers to the absence or presence of a change from the reference sequence at the position indicated. The shading, or colors red and green, refer to increased resistance or increased susceptibility, respectively. Darker shading or more intense color indicates higher significance. $IC_{50}$ units are $\mu$g/ml. Data for the identification of an epitope for PG16 antibody on gp120 protein also was generated using the present methods (data not shown).

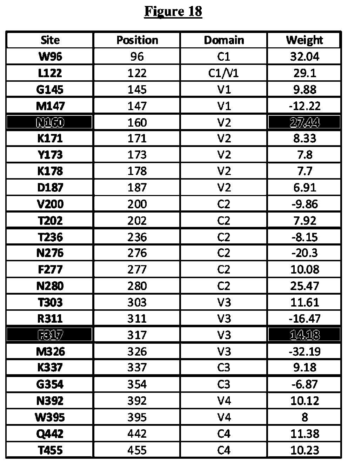

Data for the identification of an epitope for PG16 antibody on gp120 protein also was generated using the present methods (data not shown). Figure 18 is a table summarizing some information on the patches used for PG16 epitope identification on gp120 protein using the CPH model, sorted by position. Data is shown for a patch that is 12 angstroms in diameter and 2.5 angstroms deep. The "domain" column refers to commonly known functional domains within gp120. The weight column refers to the odds ratio for a residue, which identifies how significant it was.

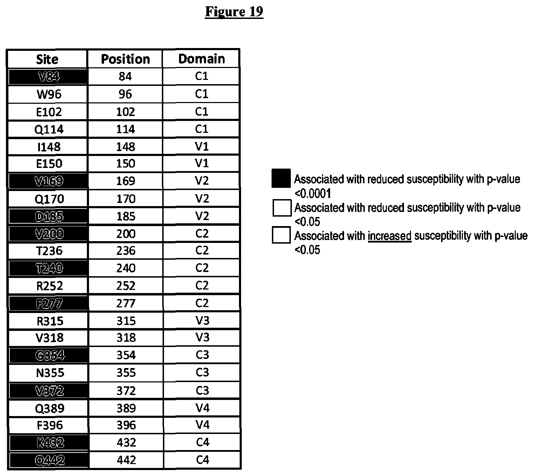

Figure 19 is a table summarizing information on the patches used for b12 epitope identification on gp120 protein. The "domain" column refers to commonly known functional domains within gp120.

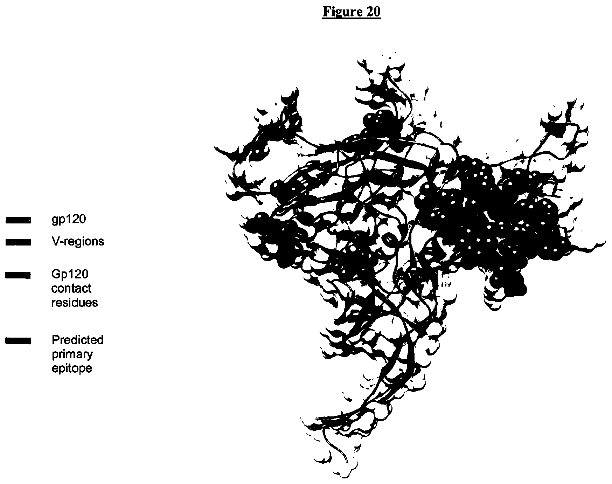

Figure 20 is a schematic representation of the b12 epitope on gp120 protein identified by embodiments of the methods of the present invention. 2NYC model of gp120 was used for visualization. The V region loops are shown at top left, center, and top right of the schematic. The gp120 contact residues are shown in green (or light gray), and the predicted primary epitopes are shown in red (or dark gray).

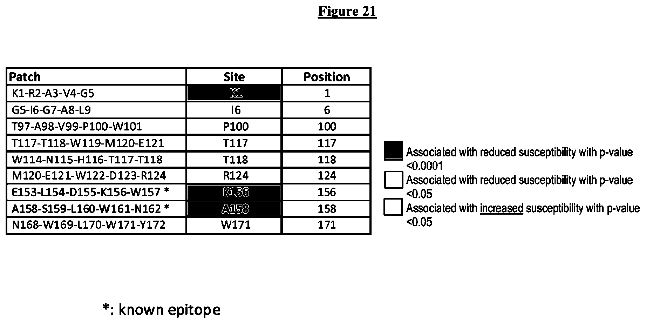

Figure 21 is a table summarizing some information on the patches used for 2F5 epitope identification on gp41 protein. Both of the known epitopes were identified with the current methods with a p-value of < 0.0001. Additional epitopes K1 and P100 were identified with the current methods with a p-value of < 0.0001 and < 0.05, respectively.

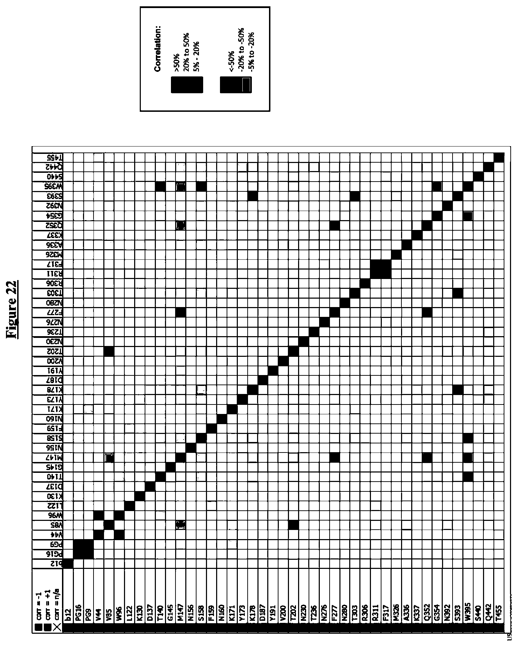

Figure 22 is a schematic representation of a co-variation matrix of the significant gp120 epitope residues identified by the methods described herein. Explained generally, blue color means that two residues vary in the same or similar way. Therefore, if one residue of the pair changes, the other is likely to also change. The stronger the blue color, the more likely this will happen. If a box is red, the two residues in that pair vary inversely. That is, if one residue changes, the other likely will not change. The stronger the color, the more likely this will happen.

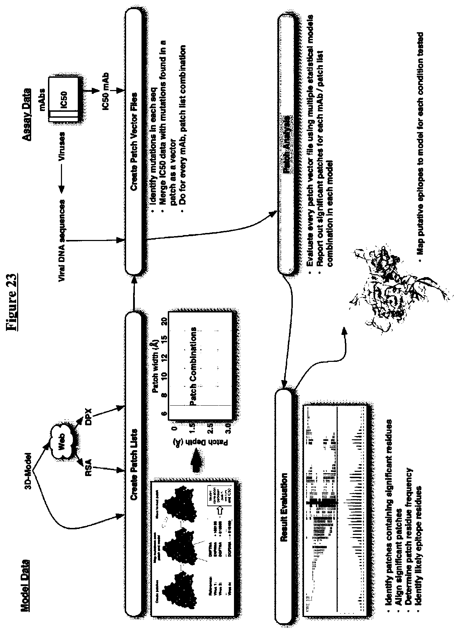

Figure 23 is a schematic representation of a method of protein binding site identification as applied to epitope prediction process.

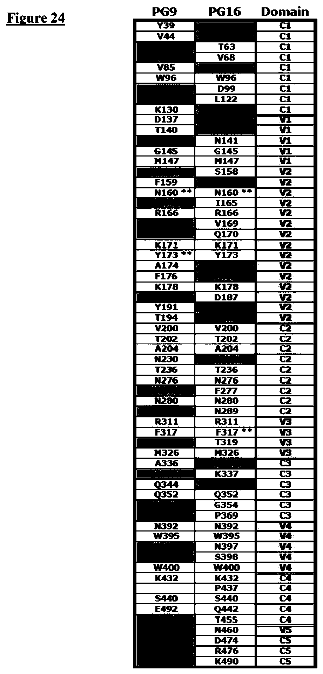

Figure 24 is a table showing a list of statistically significant (p-value<0.05) residues identified at patch thickness (dpx) of 2.5Å and surface radius of 12Å for PG9 and PG16. Highly significant (p-value<0.001) residues are marked with double asterisks (**).

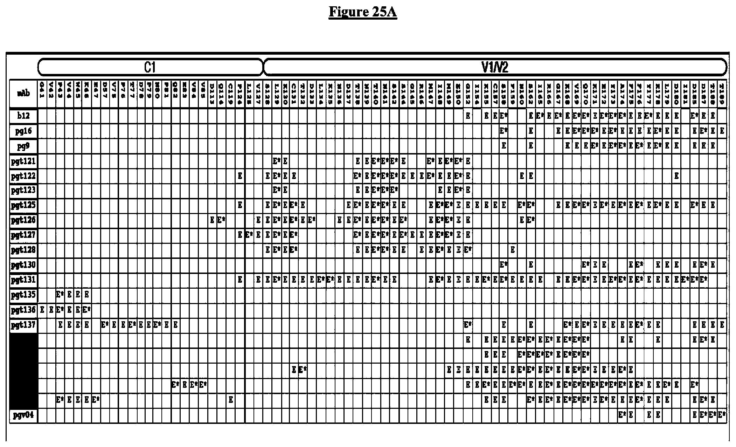

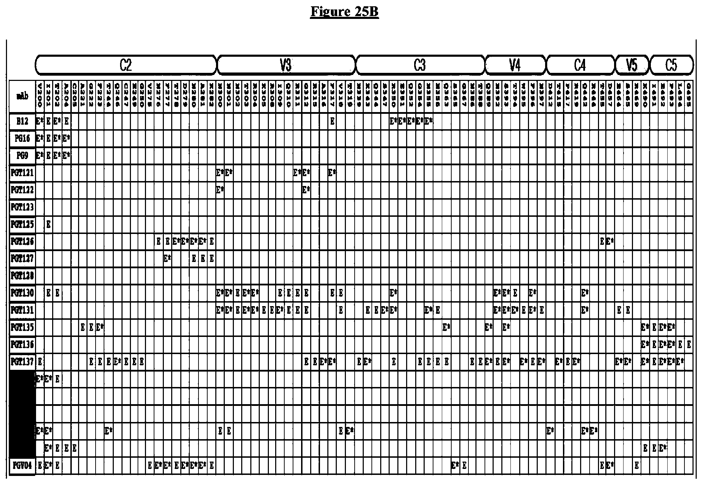

Figures 25A and 25B provide a table that schematically illustrates predicted epitope residues linearly aligned across the antibodies tested (Figure 25B is a continuation of Figure 25A). Predicted epitope residues are indicated (E) for each tested antibody. Residues found to be significant are marked (E*). The epitope residues previously identified in Walker *et al.* (2009) and Falkowska et al. ("PGV04, an HIV-1 gp120 CD4 binding site antibody, is broad and potent in neutralization but does not induce conformational changes characteristic of CD4." Journal of Virology 2012 86(8):4394-4403) are S158, S162, Y173, and F176 for pg16 and pg9; G167 for pg16; and N276, D279, S365, and T455 for PGV04. The corresponding regions of gp120 are shown across the top of the table, with constant regions indicated by a "C" and variable regions indicated by "V." Antibodies derived from the same donor are grouped together in the "mAb" column and indicated by different color or shading of that column.

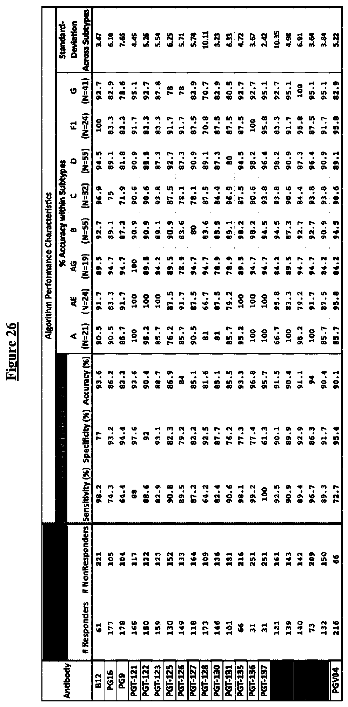

Figure 26 is a table illustrating the performance of predictive models built based on the significant sites in gp120. Antibodies derived from the same donor are grouped by different color or shading in the "Antibody" column (e.g., PG16 and PG9; PGT-121, PGT-122, and PGT-123; PGT-125, PGT-126, PGT-127, PGT-128, PGT-130, and PGT-131; PGT-135, PGT-136, and PGT-137; PGT-141, PGT-142, PGT-143, PGT-144, and PGT-145).

DETAILED DESCRIPTION

Described herein are computer-implemented methods for identification of ligand binding sites on protein molecules. One example of such a binding site is a binding site for an antibody on a protein antigen molecule, also commonly referred to as an "epitope." However, the computer-implemented methods of the present invention are broadly applicable to identification of various types of ligand binding sites on proteins, including protein-protein binding sites and protein-small molecule binding sites. Some other non-limiting examples of protein binding sites that can be identified, fully on in part, by the methods of the present invention, are binding sites for enzyme substrates, binding sites for receptor ligands, or binding sites for various medicinal molecules. The computer-implemented methods of the present invention use a combination of protein sequence data, structural information, experimental data on biological activity, and computational modeling in order to identify binding sites on protein molecules.

The terms "identify," "identification," and related terms are used, in the context of the methods described herein, to refer generally to determination of a binding site of a protein molecule. The determination can be a full or a partial determination, and can involve determination of the residues of a protein, including amino acid residues and other residues, such as carbohydrates or lipid, that are generally thought to be associated with the protein molecule. Identification can refer to prediction of potential binding sites, elucidation or clarification of a structure of known binding sites, or, generally, to any combination of these processes, or inquiry into the nature of binding sites.

It is to be understood that the terms "protein," "protein molecule," "peptide," or "polypeptide," and related terms, can refer broadly to amino acid chains, composed of standard twenty amino acids as well as non-standard amino acids, and may also include additional non-amino acid, atoms, molecules, or residues, covalently or non-covalently attached. For example, proteins can be modified *in vivo* by posttranslational modifications that attach various functional groups, such as lipids or carbohydrates, or modify the amino acids in the polypeptide chain. Proteins can also be artificially modified. Proteins can also have non-peptide groups or atoms attached, which can be called prosthetic groups or cofactors.

Protein Binding

The term "binding site" typically refers to an area of a protein molecule that interacts or "binds" with another molecule or a part of another molecule, termed a "ligand." While the term "ligand" is often used to refer to molecules that are relatively smaller in size, as compared to the protein molecule, the term "ligand" as used herein is not limited by a molecule's size. Specifically, the term "ligand" is not limited to small or relatively smaller molecules, as compared to the protein molecule or molecules, and encompasses generally all molecules or parts of molecules that participate in binding interactions with protein molecules.

In reference to protein molecules, the term "binding site" refers generally to areas of a protein tertiary structure that contribute to the specificity and affinity of the ligand-protein binding. A binding site includes residues or atoms with which a ligand molecule interacts through various non-covalent interactions, including ionic interactions, electrostatic (ionic) interactions, hydrophobic interactions, hydrogen bonding, and Van der Waals interactions. A binding site also may include residues or atoms that are not directly involved in ligand interactions, but contribute to such interactions indirectly, contribute to the conformation of the binding site, or are otherwise involved. A binding site includes a surface area of the protein, but may also include areas that are distant from the surface. The same, similar, or overlapping binding sites can have affinity to more than one ligand.

The term "affinity" generally refers to the degree of an attractive force (originating from a totality of various non-covalent interactions) between the protein and a ligand. Protein-ligand interactions are typically viewed as equilibrium phenomena, which are described as the association and dissociation reactions between a ligand and a binding site, and are characterized by the so-called "dissociation constant" that reflects a degree of dissociation between the ligand and the binding site at equilibrium. Dissociation constants are typically expressed in units of concentration, with the lower concentrations reflecting higher affinity between the protein and the ligand, which can be also described as tighter binding. Another way to describe affinity is to use a degree of saturation of the binding site, or the total number of binding sites that are occupied by ligands per unit time. High affinity ligands reside in the binding site longer than lower affinity ligands.

Various methods exist for measuring and describing binding affinity between a protein and its ligand. For example, the dissociation constant can be determined based on the measurements of the amount of a complex formed over a range of starting concentrations of a ligand. In the biomedical field, measures of biological activity of a particular ligand are often used as substitute measures of binding affinity. For example, if a ligand inhibits a particular biological response, a 50% inhibitory concentration, or $IC_{50}$ may be used. For enzymes, the Michaelis constant, or $K_m$, is often used as a measure of a substrate's affinity for an enzyme's active site. It is to be understood that the methods of the present invention are not limited by any particular measure of binding affinity, and can employ a variety of measures of binding affinity. The selection of a particular measure of binding affinity depends on the specific application of the methods of the present invention, the type of protein and/or ligand, the experimental data available potentially available, and other factors. It is to be understood that various combinations and types of experimental measures can be used. In some embodiments, employing more than one measure of binding affinity advantageously improves accuracy and other characteristics of the methods described herein.

Sequence variants

Embodiments of the computer-implemented methods of the invention may utilize genetic and phenotypic information on sequence variants (or mutants) of a protein being analyzed. It is to be understood that a full-length protein or its coding nucleic acid sequence need not be used. Instead, a part of a sequence variant may be used. For example, a part of a sequence variant may be incorporated into a vector, such as an expression vector, which can be used to collect the genetic and/or phenotypic information to be utilized in the embodiments of the methods described herein. Sequence variation may be found in a protein or nucleic acid sample obtained by any means known in the art for obtaining such samples. Such methods include, but are not limited to, obtaining a sample from an organism, cell, or culture. For example a sample of a viral nucleic acid can be obtained from a human individual infected with the virus. A sample can also be obtained from a culture, such as a cell or tissue culture. In some embodiments, the culture can be obtained from a laboratory. In other embodiments, the culture can be obtained from a collection, for example, the American Type Culture Collection. In another embodiment, a mutation or sequence variation can be present in a genetically modified protein or nucleic acid encoding it. Genetic modification can be performed using any suitable method. A sequence variant can be obtained by mutagenizing an organism, a cell, a tissue, virus, a genome, or a part of a genome. Any suitable method of mutagenesis can be used for this purpose. In one embodiment, the mutagenesis is essentially random. In another embodiment, the essentially random mutagenesis is performed by exposing an organism, a cell, a tissue, virus, a genome, or a part of a genome to a mutagenic treatment. Examples of essentially random mutagenic treatments include, for example, exposure to mutagenic substances (*e.g.*, ethidium bromide, ethylmethanesulphonate, ethyl nitroso urea (ENU)) radiation (*e.g.*, ultraviolet light), the insertion and/or removal of transposable elements (*e.g.*, Tn5, Tn10), or replication in a cell, cell extract, or in vitro replication system that has an increased rate of mutagenesis. See, for example, Russell et al., 1979, Proc. Nat. Acad. Sci. USA 76:5918-5922; Russell, W., 1982, "Environmental Mutagens and Carcinogens: Proceedings of the Third International Conference on Environmental Mutagens."

Site-directed mutagenesis may also be used. Site-directed mutagenesis can be performed according to conventional methods. See, for example, the relevant sections of Sambrook et al., 2001, "Molecular Cloning: A Laboratory Manual" Cold Spring Harbor Laboratory, 3d ed., NY; and Ausubel et al., 1989, "Current Protocols in Molecular Biology," Greene Publishing Associates and Wiley Interscience, NY. The site directed mutagenesis can be directed to, *e.g.*, a particular gene or genomic region, a particular part of a gene or genomic region, or one or a few particular nucleotides within a gene or genomic region. In one embodiment, the mutagenized nucleotides encode amino acid residues in the primary sequence of the protein that are adjacent to or near the residues known or suspected to interact with a ligand, or known or suspected to be mutated in sequence variants having changed affinity for a ligand, as compared to a reference sequence variant. In another embodiment, the mutagenized nucleotides encode amino acid residues that in the secondary, tertiary, or quaternary structure of the protein are adjacent to or near the residues known or suspected to interact with a ligand.

The presence or absence of a mutation or sequence variation can be detected by any means known in the art for detecting a mutation. The mutation can be detected in a gene that encodes a particular protein, or in the protein itself, *i.e.*, in the amino acid sequence of the protein. The detection of a mutation in specific nucleic acid sequences, such as in a particular region of a viral gene, can be accomplished by a variety of methods including, but not limited to, restriction-fragment-length-polymorphism detection based on allele-specific restriction-endonuclease cleavage (Kan and Dozy, 1978, Lancet 11:910-912), mismatch-repair detection (Faham and Cox, 1995, Genome Res. 5:474-482), binding of MutS protein (Wagner et al., 1995, Nucl. Acids Res. 23:3944-3948), denaturing-gradient gel electrophoresis (Fisher et al., 1983, Proc. Natl. Acad. Sci. U.S.A. 80:1579-83), single-strand-conformation-polymorphism detection (Orita et al., 1983, Genomics 5:874-879), RNAase cleavage at mismatched base-pairs (Myers et al., 1985, Science 230:1242), chemical (Cotton et al., 1988, Proc. Natl. Acad. Sci. U.S.A. 85:4397-4401) or enzymatic (Youil et al., 1995, Proc. Natl. Acad. Sci. U.S.A. 92:87-91) cleavage of heteroduplex DNA, methods based on oligonucleotide-specific primer extension (Syvanen et al., 1990, Genomics 8:684-692), genetic bit analysis (Nikiforov et al., 1994, Nucl. Acids Res. 22:4167-4175), oligonucleotide-ligation assay (Landegren et al., 1988, Science 241:1077), oligonucleotide-specific ligation chain reaction ("LCR") (Barrany, 1991, Proc. Natl. Acad. Sci. U.S.A. 88:189-193), gap-LCR (Abravaya et al., 1995, Nucl. Acids Res. 23:675-682), radioactive or fluorescent DNA sequencing using standard procedures well known in the art, and peptide nucleic acid (PNA) assays (Orum et al., 1993, Nucl. Acids Res. 21:5332-5356; Thiede et al., 1996, Nucl. Acids Res. 24:983-984).

In addition, hybridization or amplification assays may be used to detect abnormalities involving gene structure, including point mutations, insertions, deletions, and genomic rearrangements. Such assays may include, but are not limited to, Southern analyses (Southern, 1975, J. Mol. Biol. 98:503-517), single stranded conformational polymorphism analyses (SSCP) (Orita et al., 1989, Proc. Natl. Acad. Sci. USA 86:2766-2770), and PCR analyses (U.S. Pat. Nos. 4,683,202; 4,683,195; 4,800,159; and 4,965,188; "PCR Strategies" 1995 Innis et al. (eds.), Academic Press, Inc.).

Nucleic acids can be sequenced by any sequencing method known in the art. For example, the viral DNA can be sequenced by next generation sequencing methods, or other methods known in the art, such as but not limited to the dideoxy method of Sanger et al., 1977, Proc. Natl. Acad. Sci. USA 74:5463, as further described by Messing et al., 1981, Nuc. Acids Res. 9:309, or by the method of Maxam et al., 1980, Methods in Enzymology 65:499. See also the techniques described in Sambrook et al., 2001, "Molecular Cloning: A Laboratory Manual, Cold Spring Harbor Laboratory," 3d ed., NY; and Ausubel et al., 1989, Current Protocols in Molecular Biology, Greene Publishing Associates and Wiley Interscience, NY.

Protein or peptide fragments of interest can be sequenced by any sequencing method known in the art in order to yield the amino acid sequence of the protein of interest. An example of such a method is the Edman degradation method which can be used to sequence small proteins or polypeptides. Larger proteins can be initially cleaved by chemical or enzymatic reagents known in the art, for example, cyanogen bromide, hydroxylamine, trypsin or chymotrypsin, and then sequenced by the Edman degradation method.

Immune System

"Immune system" refers to the tissues, cells, and molecules involved in the totality of host defense mechanisms. According to current understanding, the immune system and associated immune responses have evolved to protect an organism, or host, against a wide variety of agents foreign to the host organism, such as pathogens, parasites, and toxins. In vertebrate animals, the immune system and responses include the evolutionary older "innate immune system," which broadly recognizes pathogens and does not provide lasting immunity, and the adaptive immune system, which has the ability to generate and memorize highly specific immune responses for targeting and eliminating foreign elements. An important feature of the adaptive immune system is its ability to produce antibodies, specialized protein molecules that bind with high affinity and specificity to its target molecules, termed antigens.

An antigen or immunogen is any molecule that can bind specifically to an antibody. Antigens can belong to various classes of molecules, including biological molecules, such as proteins, nucleic acids, or polysaccharides, and can possess various chemical structures, atomic composition, and size. While the terms "antigen" and "immunogen" are often used to refer to molecules that can induce adaptive immune response in vertebrate animals, including generation of antibodies, it should be understood that some antigens do not, by themselves, elicit antibody production. For example, the term "hapten" is often used to refer to small molecules that can be recognized by an antibody but are only able to stimulate production of anti-hapten antibodies when linked to a larger protein carrier. As used herein, the term "antigen" or "immunogen" is not limited to immunogenic antigens, or those antigens that can induce antibody production. Molecules that bind to antibodies but do not, by themselves, induce antibody production, such as haptens, are also included within the scope of the term "antigen" or "immunogen."

An antibody that is capable of binding a relatively large molecule, such as a protein, generally recognizes a region on the surface of the large molecule. This region is commonly referred to as an "antigenic determinant" or "epitope." As used herein, it is also referred to in some embodiments as a "binding site." The term "epitope" can also encompass not only the surface region of a particular protein described by specific amino acids, but more generally refers to a protein's tertiary structure that contributes to antibody binding. In some cases, changes in the primary structure (*i.e.*, amino acid sequence), or mutations, do not disrupt the epitope; in other cases, mutations can affect (increase or decrease) antibody binding.

Epitopes can be continuous or discontinuous, and also include continuous or discontinuous parts. A continuous epitope (or its part) is generally a continuous amino acid sequence of a polypeptide chain. Discontinuous epitopes (or their parts) are generally composed of amino acids from different parts of the polypeptide chain that are located in certain proximity in the protein's tertiary structure. Discontinuous epitopes can also be referred to as "conformational epitopes." Protein epitopes can have non-peptide components. For example, some protein epitopes contain sugar subunits that are attached to polypeptide chains.

An immune response to foreign elements requires the presence of B-lymphocytes (B cells) or T-lymphocytes (T cells) in combination with antigen-presenting cells (APC), which are usually macrophage or dendritic cells. The APCs are specialized immune cells that capture antigens. Once inside an APC, antigens are broken down into smaller fragments called epitopes - the unique markers carried by the antigen surface. These epitopes are subsequently displayed on the surface of the APCs and are responsible for triggering an antibody response in defense of the infection. When an APC displaying antigens (in the form of unique epitope markers) foreign to the body are recognized, B cells are activated, proliferating and producing antibodies. These antibodies specifically bind to the antigens present on the virus. After the antibody attaches, the APC engulfs the entire antigen and kills the virus. This type of antibody immune response is involved in the prevention of viral infection, such as HIV infection.

During an adaptive immune response, T cells are activated by recognizing the antigen displayed on the APC. There are two steps in the cellular immune response. The first step involves activation of cytotoxic T lymphocyte cells (CTLs) or CD8+ T killer cells that proliferate and kill target cells that specifically present antigens. The second involves helper T cells (HTL) or CD4+ T cells that regulate the production of antibodies and the activity of CD8+ cells. The CD4+ T cells provide growth factors to CD8+ T cells that allow them to proliferate and function.

Vaccines

Vaccinations and vaccines are used in disease prevention. Vaccines are immunogenic compositions for inducing an immune response against certain human pathogens. They are designed to stimulate the immune system to protect against various infections. In general, a vaccine is produced from an antigen, isolated or produced from the disease-causing pathogen, or a recombinant antigen from the disease-causing pathogen. When a vaccine or immunogenic composition is administered into an individual (e.g., injected into the bloodstream) as a preventive measure to create an effective immune response, the B cells in the bloodstream perceive the antigens contained by the vaccine or immunogenic composition as foreign or "non-self" and respond by producing antibodies, which bind to the antigens and inactivate them. Memory cells are thereby produced and remain ready to mount a quick protective immune response against subsequent infection with the same disease-causing agent. Thus, when an infective pathogen containing similar antigens as the vaccine enters the body, the immune system will recognize the protein and instigate an effective defense against infection. Since antibodies raised against peptides of a protein or against synthetic peptides corresponding to part of its sequence can in some cases bind to the naturally folded protein, it is sometimes possible to use synthetic peptides in vaccines. The field of vaccine design (sometimes referred to as "rational vaccine design") is concerned with identification of protein epitopes and synthetic peptides that can be used as epitopes, in order to generate antigens that can be successfully incorporated into vaccines.

HIV

While vaccines against some pathogens are very effective, development of effective vaccines against other pathogens remains a challenge. For example, HIV has evolved to evade adaptive immune system responses. Typically, the adaptive immune system targets the surface proteins or glycoproteins of animal viral pathogens. The HIV surface glycoprotein spikes are known to consist of a trimer of heterodimers formed of two glycoproteins, gp120 and gp41, which are formed from the precursor gp160 protein, encoded by the HIV *env* sequence. gp120 is organized into variable regions 1-5 (V1-V5) and conserved regions 1-5 (C1-C5). gp120 has a receptor site for one of the HIV target cells, CD4$^+$ T-cells. Based on the available structural information, this conserved CD4 receptor site appears to be hidden from antibody access. The variable regions of gp120 are the primary target of anti-HIV antibodies, but the *env* coding region exhibits enormous sequence variability and has a high mutation rate in the sequences encoding these regions. This high sequence variability provides for an effective mechanism for HIV to evade (escape from) the antibodies produced by the adaptive immune system. Not only are there a large variety of HIV subgroups, typically referred to as "clades," in various geographical regions, but there is also a constantly changing variety of HIV clones that circulate in an infected individual. When an individual is infected by HIV, the neutralizing antibody response to a dominant viral clone develops, but a so-called "escape variant" is selected and begins to proliferate, thus evading the adaptive immune system. When an antibody response to this variant develops, the selection of a new escape variant occurs.

In some known instances of HIV variability, lack of neutralization of the escape variants by already circulating antibodies can be explained by key amino acid changes in the known epitopes of gp120; in other known cases, epitope conservation does not ensure neutralization. Furthermore, many regions outside of the known epitopes have been shown to affect neutralization sensitivity. These observations can be compared to the observations with respect to HIV drug resistance, in which some mutations that impact resistance were found to be proximal to the active site of the drug, while others conferring resistance were not. Accordingly, meaningful identification of HIV epitopes remains an unresolved challenge.

Antibodies that can recognize many different variants of HIV, or so-called "broadly neutralizing" antibodies, have evolved to occur in some individuals. For example, HIV-1 broadly neutralizing antibodies PG9 and PG16 were isolated from an African clade A effective donor and were shown to be both broad and potent. Therefore, a safe and effective vaccine for HIV is thought to be theoretically possible, but the efforts to develop such a vaccine remain unsuccessful. A safe and effective HIV vaccine remains a primary goal in research and funding to try to curb the current HIV/AIDS epidemic. Known broadly neutralizing antibodies have been well characterized along with their epitopes. However to date, the design of effective immunogens from the available knowledge of these epitopes did not result in immunogens capable of producing effective immune responses in the recipients. All HIV vaccines developed to date have been unsuccessful in human trials because they have failed to provide immunogens that elicit neutralization responses to HIV. Current vaccine research efforts include finding and characterizing new broadly neutralizing monoclonal antibodies (mAb) and the antigens that produce them. Expanded and more detailed knowledge of the epitopes for already known antibodies, as well as the ability to quickly and accurately identify epitopes for new antibodies would be highly useful in the field of the vaccine design and development, including the subfield of HIV vaccine development. In the field of the vaccine development generally, and in the sub-field of HIV vaccine development, as one example, there is a need for methods that identify antigenic targets of antibodies along with the models that identify immunologically important residues of known epitopes that affect neutralization.

Methods of epitope identification

Finding an epitope of an antibody is a difficult task. Traditional methods include mutation scanning and Biacore binding interference assay. Computational methods have been developed to try to predict potential binding sites *a priori*, but these methods usually identify generic regions that have potential to be general binding sites, rather than specific regions associated with a protein of interest. Some of the known methods for identifying potential epitopes of neutralizing antibodies include the use of structural information in combination with experimental data on epitope mapping, obtained by various techniques, such as phage-display. However experimental methods for the mapping of epitopes are tedious, as they require testing a large number of antigens or large quantities of purified antigen. Currently known purely computational methods based on general knowledge of protein-protein interactions offer rapid prediction of monoclonal antibody (mAb) epitopes, but have limited accuracy.

One strategy for finding epitopes and other immunologically important residues is development of processes that use computational modeling and experimental data, and correlate experimental data with antibody binding an antigenic protein sequence. Desirable processes are accurate and allow for identification of potential epitopes, key immunologically important residues and their collective impact on neutralization sensitivity. Some examples of the useful features of desirable processes of epitope identification include prediction of neutralization breadth, prediction of escape variants of highly variable pathogens, such as HIV and influenza virus, and location of both epitope and other regions that affect antibody neutralization sensitivity.

Exemplary embodiments of the computer-implemented methods described herein are processes useful for identifying protein ligand binding sites such as antibody epitopes, which are also referred to as "binding site identification methods," "epitope identification methods," or "methods for epitope identification," "methods of identifying antibody epitopes," or by related phrases. Specific working examples included herein illustrate the present methods by describing identification of epitopes of HIV neutralizing antibodies. It is to be understood, however, that the computer-implemented methods of epitope identification are not limited by the specific anti-HIV antibodies or HIV antigens discussed in the working examples, or to HIV. Rather, the embodiments of the methods of the present invention illustrated by these examples are broadly applicable to identification of the epitopes of any protein. Some advantages of the embodiments of the computer-implemented methods described in this section are increased speed and accuracy of epitope identification, as compared to the previously known methods. Certain embodiments of the computer-implemented methods of epitope identification, as well as the systems for performing these methods as described herein, are also advantageous, for example, because they are used for design of commercially valuable immunogenic compositions, such as vaccines or antibodies that possess improved properties, as compared to known immunogenic compositions. Among these properties is one or more of immunogenicity, higher neutralization activity, or broader neutralization range of the antibodies induced by the immunogenic compositions, and lower probability of escape of a target pathogen from the adaptive immune response induced by the immunogenic composition.

The computer-implemented methods of identifying antibody epitopes described herein achieve unexpected and remarkable results in achieving the above goals by correlating antibody neutralization data and genotype to protein structure through the use of more than one computational modeling method in order to arrive at the most likely epitope residues. Computer-implemented methods according to embodiments of the present invention can also be applied to development of synthetic antibodies, such as therapeutic antibody drug development, including development of nanobodies, camelids and antibody-drug conjugates. The computer-implemented methods of identifying antibody epitopes described herein include methods that can be referred to as Multi-Modal Binding Site Prediction. A high-level schematic overview of the workflow of the epitope identification methods described herein is shown in Figure 5.

The computer-implemented methods of epitope identification described herein use sequence information for a plurality of sequence variants of a protein being analyzed, experimental data on a measure of affinity of the sequence variants for one or more antibodies, and the known or predicted three-dimensional model of the protein structure, in order to identify epitope residues. Computer-implemented methods of epitope identification described herein utilize greatly improved patch analysis methods and techniques, further described below.

Patch Analysis

The computer-implemented methods of protein binding site identification described herein employ greatly improved patch analysis techniques. Improved patch analysis techniques are included within the scope of the embodiments of the present invention. "Patch analysis" is a technique that combines data on protein structure, sequence data, and phenotypic experimental data on binding affinity of protein sequence variants (mutants) in order to identify the areas of a protein molecule that contribute to binding of the protein to its ligand. Previously used patch analysis is described, for example, in Zhang et al. ("Prediction of conformational B-cell epitopes from 3D structures by random forest with a distance-based feature." BMC Bioinformatics, 2011, 12:341). The greatly improved patch analysis described herein defines areas on the protein structure termed "patches," which contain a number of residues within close proximity of each other, and correlates the sequence variability within each patch with the experimental data on protein-ligand affinity for each sequence variant. For example, the embodiments of the methods of the present invention that relate to epitope identification can use sequence variability within the patches of a protein antigen as well as antibody neutralization data obtained for each sequence variant.

In the improved patch analysis techniques described herein, the data on sequence variation within each patch and protein-ligand affinity for each sequence variant is compiled as a vector or array data file. The sequence variation within each file is presented as a series of binary values, as discussed in more detail below. For the protein-ligand affinity values that are not binary, the actual values can be used. Thus, in some instances, the data file can be a hybrid of both non-binary and binary values presented as a vector file. For example, in a vector file identified as an array of 5 values [15.3,0,0,1,1], the first vector value is the protein-ligand affinity value, such as $IC_{50}$ value, while the remaining four positions are binary values representing the sequence variation within the patch. In some other instances, the protein-ligand affinity value can be converted into binary form. Binary or non-binary form of protein-ligand affinity value representation is selected based on the computational method to be applied to the field. Computational methods are applied to the resulting vector files in order to test each patch for an ability of the sequence variation within the patch to predict changes in protein-ligand affinity.

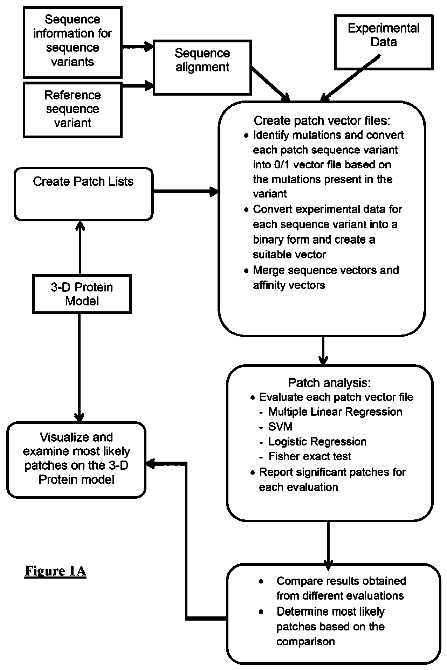

Generally, the improved patch analysis techniques employed in the computer-implemented methods of the present invention evaluate each patch vector file according to more than one (meaning a plurality, several, two or more, three or more, four or more, five or more) computational methods, which can be also described as computational models, techniques, or procedures, bioinformatics methods, models, techniques, or procedures, or by other related terms used in the field of computational modeling. The improved patch analysis according to some embodiments of the methods of the present invention uses at least one method that involves multivariate analysis, at least one method that involves univariant analysis, and at least one machine learning algorithm. However, other types and combinations of the computational methods can be used. The improved patch analysis techniques compare the results obtained by several computational methods to identify the binding site candidates. Variations of the improved patch analysis techniques according to certain embodiments of the present invention can use patches of variable depths, as discussed further below. Some of the features of the improved patch analysis are schematically illustrated in Figure 1A.

Patches are defined as groups of residues that are in close proximity to each other based on an available three-dimensional model of a protein structure. The three-dimensional structure models used for patch definition can be derived using the techniques of structural biology, such as X-ray crystallography, determined by computer modeling, or by any combination of experimental and computational techniques. Generally, selection of the protein model employed in various embodiments of the methods of the present invention takes into account one or more of the following considerations. Preference is given to models approaching involvement of the full-length protein. Higher resolution models are preferred.

Models that contain fusions with extraneous proteins must have those fusion portions removed prior to patch generation. Predicted structural features of a model can be acceptable. For example, predicted structures can be used to overcome lack of definition in the flexible protein regions, such as the so-called "loop clipping" that commonly is observed in the models based on the X-ray crystallographic data, as illustrated in Figure 6. Structural information from several different three-dimensional structures or models can be used to obtain a three-dimensional structure suitable for patch definition or patch analysis generally.

With respect to the improved patch analysis described herein, close proximity of patch residues within a protein structure means residues whose $\alpha$-carbon is located at a pre-defined distance or distances from the $\alpha$-carbon of the pre-selected central amino acid residue of the patch and where the average atomic depth of all of the atoms in a residue does not exceed the specified depth threshold. This amino acid residue can be termed as patch "center," and its $\alpha$-carbon as patch "center point."

Solvent-accessible residues within a pre-defined "surface radius" from the center point are included within a patch. Surface accessibility of amino acid residues to a solvent is calculated using available software, one example of which is the DSSP software application, commonly used in a wide variety of computational macromolecular structure research fields. This method uses that value (ASA) to calculate a normalized Relative Surface Area (RSA) (Singh and Ahmad, BMC Structural Biology 2009, 9:25). A cutoff for "surface accessibility" is assumed based on conventional criteria. For example, residues with less than 20% of their surface accessible to solvent can be considered buried. The term "surface radius," as used in certain embodiments of the methods of the present invention can be defined as about 20 to about 5 angstroms, about 20 to about 10 angstroms, about 15 to about 5 angstroms, about 10 to about 5 angstroms, about 20 to about 15 angstroms, about 20 to about 19 angstroms, about 19 to about 18 angstroms, about 18 to about 17 angstroms, about 17 to about 15 angstroms, about 15 to about 12 angstroms, about 12 to about 10 angstroms, about 10 to about 8 angstroms, about 8 to about 7 angstroms, about 7 to about 6 angstroms, or about 6 to about 5 angstroms. Some examples of "surface radius" are 6, 8, 10, 12, 15, and 20 angstroms. When patches are generated, a single radius at a time is used. In certain embodiments, multiple patches can be tested, each using a different radius.

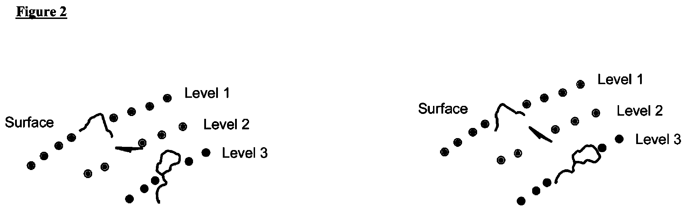

The computer-implemented methods of the present invention may use patches of variable "thickness" (or "thinness"). These and related terms are used, in the context of patch analysis, to refer to the depth radius from the patch center point. The concept of Residue Interaction Networks (RINs), generally described in Zhang *et al.* (2011). In general terms, the concept of RINs accounts for the fact that protein molecules are not rigid, static structures. RINs represent groups of amino acids within a protein that interact over relatively large distances. Within the RINs concept, internal residues may contribute to defining a functional epitope. RINs are schematically illustrated in Figure 2. In addition to the surface residues within a pre-defined radius from a patch center-point, "thick" patches include non-surface residues within a defined depth radius of that center point.

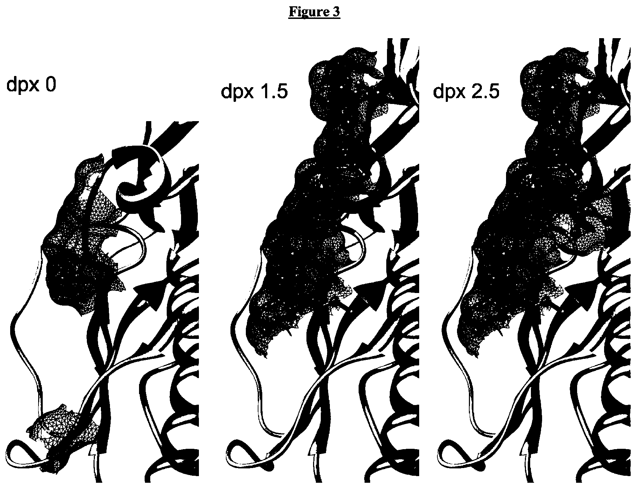

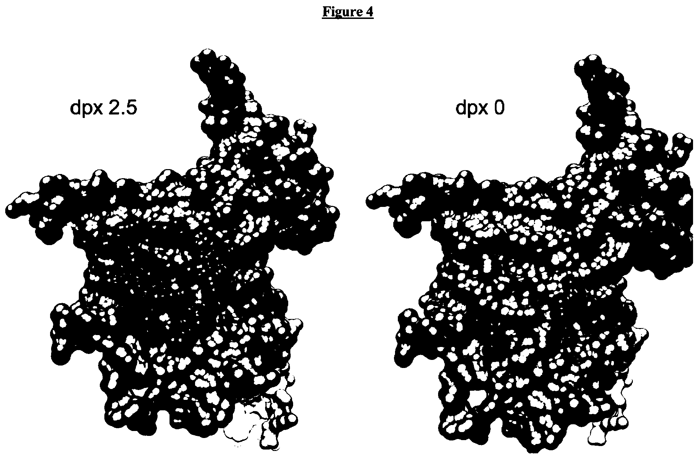

For some embodiments of the computer-implemented methods of the present invention, mean residue atomic depth (dpx), which can also be referred to as "atom depth," is used as a way of describing "thick surface patches;" dpx is typically defined as the closest distance, in angstroms, of a non-hydrogen buried atom from its closes solvent-accessible protein neighbor. The concept of atom depth is described, for example, in Pintar et al. "Atom depth as a Descriptor of protein interior" Biophysical Journal, 2003, 84:2553-2561. Within the dpx concept, as used in various embodiments of the methods of the present invention, solvent-accessible atoms have dpx defined as 0. All buried atoms have dpx >0, with the larger distances from the surface corresponding to higher dpx values. The methods of the present invention can also use the concept of "layers," also described in Pintar *et al.* (2003) In this concept, buried protein atoms are described as distributed in discrete layers with respect to the protein surface (dpx =0), with the first layer defined at approximately 1.50 dpx deep from the protein surface and the second layer defined at approximately 2.50 dpx deep from the protein surface. Accordingly, the patches selected for a particular variation or stage of the present methods can be a "surface patch," with dpx =0, a "first layer" patch, with dpx = 1.5, or a second layer patch with dpx = 2.5. These patches are schematically illustrated in Figures 3 and 4. In certain embodiments, the dpx may be about 3.0.

In the improved patch analysis techniques used in the computer-implemented methods of the present invention, several computational methods are used to model the relationship between occurrences of variation in protein sequence, or mutations, within each patch and the phenotypic measurements characterizing the affinity between the protein sequence variants and a ligand. Sequence variations for each amino acid in each sequence variants and each patch are represented in binary form and captured as vectors of 0 or 1, with 0 meaning no mutation and 1 meaning a mutation at a particular amino acid residue. A protein sequence variant with strong binding affinity is used as a reference variant. The information on binding affinity of each sequence variant is also captured both as a raw value and in a binary form and compiled as a vector file for use with some analysis methods as described above. For example, in some cases, 1 can be used to reflect that a change in affinity from the reference variant is present, 0 if there is no change in affinity. However, other binary representations can be employed, based on the computational method to be used. As a result of the above binary data capture, a set of vectors is generated that reflects affinity properties of each sequence variant. A set of vectors is also generated for each patch, based on the mutations present in each sequence variant, and this vector set is merged with the "affinity vector set," thus creating a "patch vector file," in which the amino acid sequence of a protein variant is related to the affinity data.

Patch analysis maps mutation patterns on each patch and examines this patch's ability to explain binding affinity by using various computational techniques. In the present methods, for each patch, multiple computational techniques are used to analyze the relationship between mutations within the patch and binding affinity. One computational technique that can be suitably employed in the embodiments of the methods of the present invention is Multiple Regression Analysis (MLR). Other suitable computational techniques are Logistic Regression (LR), Support Vector Machine (SVM), and Fischer's Exact test (FET). In some embodiments, the computational methods may include, but are not limited to, Hidden Markov Models, Neural Networks, Random Forest, Decision Trees, or Bayesian Networks.

Key potential binding site residues are identified as residues the variation at which exhibits statistically significant correlation with the observed phenotypic changes in binding affinity. The results of the different computational methods are compared in order to identify the most likely key residues that contribute to the binding site. For example, the residues that are identified as significant by more than one computational technique are considered more likely to be a part of a binding site than the residues identified in only one technique. These key residues are then used to identify their parent patches, termed here "significant patches."

The residues comprising these significant patches are then arranged spatially along a linear continuum, one below the other as illustrated in Figures 10A-10C, by position, so that for example, all of the E208 residues from each patch are in a vertical column. Then each column is evaluated to determine the frequency of occurrence that each position occurs within the group of patches as a whole. Residues that have a very high to high frequency are considered highly probable to be contributing to the binding site. Residues with a low to medium frequency are considered less likely contributing epitope residues. Improved patch analysis techniques described herein can use patches of varying parameters, such as patches with the same center but different surface radii and thickness to improve identification of binding site residues. Patches with several thresholds for surface radius and depth can be generated, and systematically tested for their ability to predict ligand binding. The residues that occur with higher frequency under varying patch parameters are considered to be more likely to contribute to the binding site.

Computer-implemented methods of protein binding site identification according to the embodiments of the present invention can use data from different ligands of the same type to locate the regions that are most likely to be involved in binding of a particular family of ligands. For example, information on binding of several different antibodies to a protein can be used to identify to key protein regions that are most likely to be involved in antibody binding. These regions can be further investigated as potential vaccine immunogens.

In some embodiments, the methods further include the steps of producing a vaccine or other immunogenic composition based on the binding site identified by the present methods and/or administering to an individual a vaccine or other immunogenic composition based on the binding site identified by the present methods in order to confer upon the individual the ability to mount an immune response upon exposure to the protein, or cell or organism that comprises the protein.

In other embodiments, the methods further include the steps of producing an antibody based on the binding site identified by the present methods and/or administering to an individual the antibody in an amount that is effective to disrupt the interaction of the protein with another protein or small molecule ligand.

Vaccine or Other Immunogenic Compositions

Vaccines or other immunogenic compositions may be produced based on the binding sites identified by the present methods. The vaccine or other immunogenic composition may comprise one or more immunogens (e.g., as identified by the present methods and or previously identified as an immunogen) as the active ingredient in combination with one or more additional components, such as a pharmaceutically acceptable excipient, carrier, buffer, stabilizer, or other materials well known to those skilled in the art. Such materials should be non-toxic and should not interfere with the efficacy of the active ingredient. The precise nature of the carrier or other material may depend on the route of administration. The phrases "pharmaceutically or pharmacologically acceptable" refer to molecular entities and compositions that do not produce an adverse, allergic, or other untoward reaction when administered to an animal, or a human, as appropriate. As used herein, a "pharmaceutically acceptable carrier" includes any and all solvents, dispersion media, coatings, antibacterial, and antifungal agents, isotonic and absorption delaying agents, and the like. The use of such media and agents for pharmaceutically active substances is well known in the art. Except insofar as any conventional media or agent is incompatible with the active ingredient, its use in the therapeutic compositions is contemplated. For human administration, preparations should meet sterility, pyrogenicity, and general safety and purity standards as required by FDA Office of Biologics standards. Supplementary active ingredients can also be incorporated into the compositions. The techniques of preparation of the immunogenic compositions are generally well known in the art as exemplified by Remington's Pharmaceutical Sciences, 16th Ed. Mack Publishing Company, 1980.

As used herein, the term "administering" refers to various means of introducing a composition into a cell or into an individual. These means are well known in the art and may include, for example, injection; tablets, pills, capsules, or other solids for oral administration; nasal solutions or sprays; aerosols or inhalants; topical formulations; liposomal forms; and the like. As used herein, the term "effective amount" refers to an amount that will result in the desired result and may readily be determined by one of ordinary skill in the art.

In certain aspects that do not form part of the invention, the composition is to be administered via injection, and the preparation of an aqueous composition that contains the immunogen(s) as an active ingredient will be known to those of skill in the art. Typically, such compositions can be prepared as injectables, either as liquid solutions or suspensions; solid forms suitable for using to prepare solutions or suspensions upon the addition of a liquid prior to injection can also be prepared; and the preparations can also be emulsified. The pharmaceutical forms suitable for injectable use include sterile aqueous solutions or dispersions; formulations including sesame oil, peanut oil, or aqueous propylene glycol; and sterile powders for the extemporaneous preparation of sterile injectable solutions or dispersions. In all cases, the form should be sterile and fluid to the extent that syringability exists. It should be stable under the conditions of manufacture and storage and should be preserved against the contaminating action of microorganisms, such as bacteria and fungi.

The disclosed compositions can be formulated into a sterile aqueous composition in a neutral or salt form. Solutions as free base or pharmacologically acceptable salts can be prepared in water suitably mixed with a surfactant, such as hydroxypropylcellulose. Pharmaceutically acceptable salts, include the acid addition salts (formed with the free amino groups of the protein), and those that are formed with inorganic acids such as, for example, hydrochloric or phosphoric acids, or such organic acids as acetic, trifluoroacetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine, and the like.

Suitable carriers include solvents and dispersion media containing, for example, water, ethanol, polyol (for example, glycerol, propylene glycol, and liquid polyethylene glycol, and the like), suitable mixtures thereof, and vegetable oils. In many cases, it will be preferable to include isotonic agents, for example, sugars, or sodium chloride. The proper fluidity can be maintained, for example, by the use of a coating, such as lecithin, by the maintenance of the required particle size in the case of dispersion and/or by the use of surfactants.

Under ordinary conditions of storage and use, all such preparations should contain a preservative to prevent the growth of microorganisms. The prevention of the action of microorganisms can be brought about by various antibacterial and antifungal agents, for example, parabens, chlorobutanol, phenol, sorbic acid, thimerosal, and the like. Prolonged absorption of the injectable compositions can be brought about by the use in the compositions of agents delaying absorption, for example, aluminum monostearate, and gelatin.

Prior to or upon formulation, the present immunogenic compositions should be extensively dialyzed to remove undesired small molecular weight molecules, and/or lyophilized for more ready formulation into a desired vehicle, where appropriate. Sterile injectable solutions are prepared by incorporating the active agents in the required amount in the appropriate solvent with various of the other ingredients enumerated above, as desired, followed by filter sterilization. Generally, dispersions are prepared by incorporating the various sterilized active ingredients into a sterile vehicle that contains the basic dispersion medium and the required other ingredients from those enumerated above.

In the case of sterile powders for the preparation of sterile injectable solutions, the preferred methods of preparation are vacuum-drying and freeze-drying techniques that yield a powder of the active ingredient, plus any additional desired ingredient from a previously sterile-filtered solution thereof.

Systems

Systems are provided for carrying out the methods of identification of a protein binding site described herein. Embodiments related to the systems comprise a computer-readable medium on which is encoded programming code for the computational processes employed in the methods described herein. Embodiments of a system may comprise a system comprising a processor in communication with a computer-readable medium, the processor configured to perform the methods described herein. Suitable processors and computer-readable media for various embodiments of the present invention are described in greater detail below.

Thus, a system for identification of a ligand binding site of a protein is provided, comprising a computer readable medium; and a processor in communication with the computer readable medium, the processor configured to apply suitable computational processes used in the methods. The processor may, in certain embodiments, be further in communication with a database comprising data for a plurality of protein sequences, where the processor is configured to compare the nucleic acid and/or amino acid sequence of a protein being analyzed according to the data in the database, in order to determine if there is a mutation in the sequence of the protein being analyzed.

In other embodiments, a computer readable medium is provided, on which is encoded program code for identification of a binding site of a protein, the program code comprising code configured for applying computational methods for correlating the sequence variability within areas of a protein structure with the experimental data on the affinity of each sequence variant for a ligand, and testing each area for an ability of the sequence variability within the area to predict changes in the affinity. In certain embodiments, the programming code comprises code configured for using a three-dimensional model of the protein's structure to define areas on the protein structure containing a plurality of residues within close proximity of each other.

Embodiments of the systems for carrying out the methods of identification of a protein binding site can be implemented in digital electronic circuitry, in computer hardware, firmware, software, or in combinations thereof. In one embodiment, a computer may comprise a processor or processors. The processor may comprise, or have access to, a non-transitory computer-readable medium, such as a random access memory coupled to the processor. The processor may execute computer-executable program instructions stored in memory, such as executing one or more computer programs including a sampling routine, a routine for the identification of a ligand binding site by patch analysis as disclosed herein, and suitable programming to produce output to generate the analysis described in detail herein.

Such processors may comprise a microprocessor, a digital signal processor (DSP), an application-specific integrated circuit (ASIC), field programmable gate arrays (FPGAs), and state machines. Such processors may further comprise programmable electronic devices such as PLCs, programmable interrupt controllers (PICs), programmable logic devices (PLDs), programmable read-only memories (PROMs), electronically programmable read-only memories (EPROMs or EEPROMs), or other similar devices.

Such processors may comprise, or may be in communication with, media, for example tangible computer-readable media that may store instructions that when executed by the processor, can cause the processor to perform the steps described herein as carried out, or assisted, by a processor. Embodiments of computer-readable media may comprise, but are not limited to, all electronic, optical, magnetic, or other storage devices capable of providing a processor, such as the processor in a web server, with computer-readable instructions. Other examples of media comprise, but are not limited to, a disk, CD-ROM, magnetic disk, memory chip, ROM, RAM, ASIC, configured processor, all optical media, all magnetic tape or other magnetic media, or any other medium from which a computer processor can read. Also, various other devices may include computer-readable media, such as a router, private or public network, or other transmission device. The processor, and the processing may be in one or more structures, and may be dispersed through one or more structures. The processor may comprise code for carrying out one or more of the methods (or parts of methods) described herein.

The system may comprise a data compiling system as well as a means for the user to interact with the system as the analysis proceeds. Thus, in certain embodiments, the present systems may comprise a system for collecting and/or compiling data from a plurality of assay measurements and/or sequencing data and transmitting the data to a computer, and a system for transmitting the results of the analysis to a user. The systems may be designed for high-throughput analysis of DNA and/or amino acid sequencing data, or for high throughput affinity analysis.

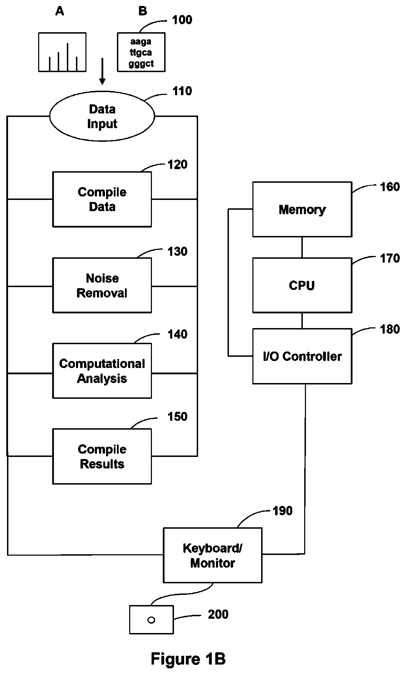

Figure 1B schematically illustrates the flow of information in an exemplary system for carrying out the methods according to certain embodiments of the present invention. As discussed above, a computer processor or CPU may include, for example, digital logic processors capable of processing input, executing algorithms, and generating output as necessary in response to the inputs received from the touch-sensitive input device. As detailed herein, such processors may include a microprocessor, such as an ASIC, and state machines, and/or other components. Such processors include, or may be in communication with, media, for example computer-readable media, which stores instructions that, when executed by the processor, cause the processor to perform the steps described herein.

Thus, in an embodiment, the starting point may comprise data **100** generated from a database of assays for binding affinity **100A** and amino acid or nucleic acid sequences of protein sequence variants **100B.** Once the data has been collected **110,** it may be compiled **120** and/or transformed if necessary using any suitable software. It is to be understood that the terms "compile," "compilation," and related terms are used, in the context of data manipulations, to refer to any or all of the steps used for converting the data (such as affinity data and sequence data) into a form suitable for analysis by the computational techniques employed in the methods and systems according to the embodiments of the present invention. For example, information on the sequence variability within each area and the data on affinity for each sequence variant can be represented in a binary form and compiled as a vector file. Both the step of representing data in a binary form and compiling it as a data file can be referred to as "compilation," separately or collectively, and any other suitable data manipulation steps can be included. The data may be stored in the computer memory **160** and used as required.

At each point in the analysis, the user may input instructions via a keyboard **190,** disk, remote access (*e.g.*, via the internet) **200,** or other access means. The user may enter instructions including options for the run, how reports should be printed out, and the like. Also, at each step in the analysis, the data may be stored in the computer using a storage device common in the art such as disks, drives, or memory **160.** As is understood in the art, the processor **170** and I/O controller **180** are required for multiple aspects of computer function. Also, in certain embodiments, there may be more than one processor.

The data may also be processed to remove noise **130.** In some cases, the user, via the keyboard **190,** disk, or remote access **200,** may want to input variables or constraints for the analysis, as for example, the threshold for determining noise.

In the next step, computational analysis is performed according to the methods described herein **140.** The results of the analysis may then be compiled and provided in a form for review by a user **150.**

EXAMPLES

Embodiments of the present methods and systems are illustrated by the following examples, which are not to be construed in any way as imposing limitations upon the scope thereof. During the studies described the following examples, conventional procedures were followed, unless otherwise stated. Some of the procedures are described below for illustrative purpose. Any examples not falling within the scope of the claims are provided for illustrative purposes only.

EXAMPLE 1

*Virus selection*

A virus panel containing 264 clonal HIV-1 viruses of diverse clades was used in epitope identification described in Examples 7 and 8. A virus panel containing 282 clonal viruses collected from unrelated patients was used in epitope identification methods described in Example 9. The viruses in both panels were of diverse subtypes and recombinants, and included virus clones from each of the subtypes A through J.

EXAMPLE 2

*Neutralization assay and experimental data representation*

A recombinant virus assay involving a single round of virus infection was used to measure neutralization. The assay is generally described in Richman et al. Proc. Natl. Acad. Sci. 2003, 100(7):4144-49. Briefly, the HIV *env* gene libraries were obtained by PCR amplification from the source material and cloned into the *env* expression test vector. The resulting *env* expression vectors were co-transfected with an env-deleted genomic vector containing a luciferase reporter. The resulting "pseudotyped" viruses were pre-incubated for 1 hour with serial dilutions of antibody and then used to infect U87 cells that express CD4 plus the CCR5 and CXCR4 co-receptors (U87/CD4/CCR5/CXCR4). Neutralizing activity was expressed as the percent inhibition of viral replication (luciferase activity) at each antibody dilution or concentration compared to a control culture without antibody. The antibody concentration at which 50% inhibition ($IC_{50}$) was achieved was determined and expressed as the reciprocal of the plasma dilution or antibody concentration conferring 50% inhibition. Figure 7 is a schematic representation of an example of experimental data related to $IC_{50}$ determination. The $IC_{50}$ value is converted to 0/1 when doing SVM, logistic regression, and univariate analysis. In some cases, more than one binary representation was created in order to suit different computational methods. The resulting binary data was compiled in a vector file.

EXAMPLE 3

*env gene sequencing and sequence analysis and representation*

The sequences of the *env* coding region (gp160) sequence variants of the *env* expression vectors constructed for the neutralization assay were determined using a conventional dideoxy chain-terminator sequencing kit (ABI, Foster City, CA). A set of sixteen primers was used to generate overlapping and redundant sequences from both DNA strands. The nucleotide sequences obtained were computationally translated into amino acid sequences, and the resulting amino acid sequences were aligned to the gp160 sequence of the laboratory reference strain HXB2 (GenBank accession number K03455). Due to the frequent occurrence of mixtures of viruses containing insertions or deletions in the variable regions of gp120, most *env* sequences contained regions of ambiguity. Sequence alignments were performed using the profile Hidden Markov Model (HMM) software HMMER. The profile HMM was previously constructed from 291 cross-clade gp160 sequences. Amino acid positions were numbered using the official HXB2 sequence available from Los Alamos National Laboratory (http://www.hiv.lanl.gov/). A binary representation of each patch for each sequence variant was obtained using suitable software (custom written but suitable) to compare each amino acid position in each patch to the amino acids at the same positions in the sequence variants. Amino acids identical to HXB2 at a given position were coded as 0, and amino acids different from HXB2 at a given position were coded as 1. Insertions and deletions were reported as 1 at the HXB2 position preceding the insertion or gap. Vector files representing each patch for every sequence were created.

EXAMPLE 4

*Protein model selection and modification*

More than 24 crystal protein structures publicly available as pdb files and containing gp120 protein were considered. 3JWD, a crystal structure of an HIV-1 gp120 core with intact gp41-interactive region in its CD4-bound state, was used for model selection. However, the 3JWD model contains truncated regions representing variable loops V1-V3. Therefore theoretical structural predictions of the full-length HxB2 gp120 molecule were made using the program CPHModels 3.2 The quality of the predicted model was assessed by creating an alignment of the $\alpha$-carbon backbones of 3JWD and the prediction using the DaliLite server. Several predicted gp120 models were compared to the 3JWD structure. The CPH model construct showed minor overall deviation through the core gp120 region with a root-mean-square deviation (RMSD) of 1.1. Although the structures of the loop regions in the CPH model is known to be unreliable, this was not expected to interfere with the epitope determination, since the rotational freedom in the CPH model was constrained to the defined loop regions. The CPH model and 3JWD model are schematically shown in Figure 6. Both the 3JWD and CPH models were used for patch analysis of gp120 protein described in Example 7. Patch analysis for gp41 described in Example 8 used the known model 1ENV, based on an X-ray crystal structure determination. The first thirty amino acids were removed, where known to be a GCN4 fusion. The patch analysis described in Example 9 used the CPH model.

EXAMPLE 5

*Patch Definition*

At least some of the following considerations were taken into account when defining the patches. In known protein modeling methods, with respect to the surface residues, the highest solvent accessibility (ASA) conformation is usually assumed based on Ala-X-Ala or Gly-X-Gly observation. In the present methods, a method described in Singh et al., BMC Structural Biology 2009, "Context dependent reference states of solvent accessibility derived from native protein structures and assessed by predictability analysis" was used, which employs highest observed ASA (HOA), rather than highest possible ASA, in order to obtain a more accurate determination of surface accessibility. The method of Singh *et al.* was applied to calculations of HOA for gp120, with the modification that Relative Surface Area (RSA) instead of standard surface area was used, which was calculated as RSA=ASA/HOA.

In the subsequent iterative analysis, each solvent accessible residue of a protein (residues with an RSA $\geq 0.2$) was considered the center of a patch. Multiple sets or "lists" of patches were generated in some instances. For example, a set of patches of varying surface radius (6 angstroms, 8 angstroms, 10 angstroms, 12 angstroms, 15 angstroms, 20 angstroms) was generated in some cases. All residues whose $\alpha$-carbons were within the radius from the $\alpha$-carbon of the patch center and which had an ASA value indicating they were on the surface were included within a patch. In some instances, sets of patches of varying thickness (e.g., dpx = 0, dpx = 1.5, dpx = 2.5, dpx = 3) were generated for each surface radius. The mean residue depth for each gp120 model structure was calculated according to the method of Pintar *et. al.* using the tool provided at the DPX server with a sphere radius of 1.4 angstroms.

Some residues were members of multiple patches. Patches comprising all the same residues were merged into a single instance to remove duplicates. Patch vector files, which represented the data for each patch and each sequence variant in a binary form, were generated using suitable software. The binary vector representations of the sequence data and $IC_{50}$ data for each sequence variant were combined.

EXAMPLE 6

*Data mining*

A combination of several machine learning and statistical models was employed to perform data mining on pairs of sequences and $IC_{50}$ data in order to identify significant associations between presence or absence of mutation and changes in $IC_{50}$, as compared to the reference virus. A combination of multivariate analysis methods, univariate analysis methods, and a machine learning algorithm was used.

One multivariate analysis employed was Multiple Linear Regression (MLR), which modeled the relationship between occurrence of a mutation in a patch (independent variables) and the log-transformed $IC_{50}$ value (response variable). Changes in $IC_{50}$ from the reference virus HXB2 were captured in a binary form (1 if present; 0 if not present). A conventional 10-fold cross validation was applied; specifically, the model was built using 9/10ths of the dataset and tested on the remaining 1/10th of samples. For the 10 test sets taken together, the agreement between measured and predicted $IC_{50}$s was calculated by their F statistic, and the resulting p-value was corrected for repeated sampling using the Bonferroni method. A patch score calculated for each patch was equal to: 1 - [$\log_{10}$ (corrected p-value of patch$_i$)], where i is the position of the central residue of the patch. All patches were ranked according to their significance score, and those with a score > 2.3 (*i.e.*, p-value < 0.05) were selected as significant predictors. Regression models were generated by the function lm (R statistical package version 2.2.1).

Another multivariate analysis method for correlating the neutralization response as a binary measurement with the mutation vectors was Logistic Regression (LR). For LR, binary representation of the existence of neutralization response in a sequence variant was used (0 if the neutralization response is present, 1 if not).

A machine-learning algorithm, Support Vector Machine (SVM) technique, was also used. For SVM, the binary representation of the neutralization response used was the existence of the response (-1) versus no-response (1) in a particular sequence variant.

Fisher's Exact test was also utilized as a univariate analysis technique calculating odds-ratio and statistical significance (p-value) in order to identify significant associations between the presence or absence of a mutation with a neutralization response.

A software application was created and implemented to automate both the comparison of patches and their mapping onto the three-dimensional protein structure.

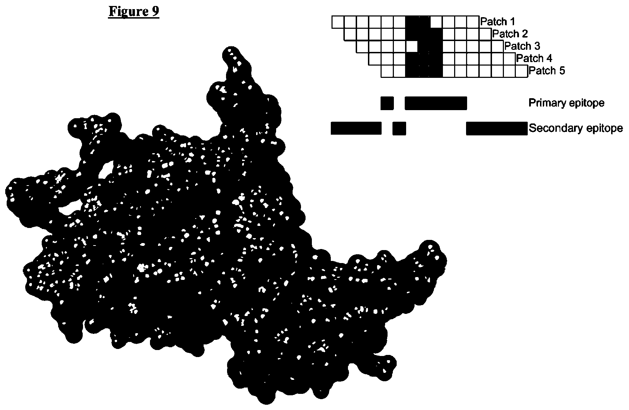

The above methods were applied to all sets of patches for a particular monoclonal antibody. MLR used the $IC_{50}$ value as a continuous measurement, and logistic regression, svm, and Fisher's test used it as a binary variable ($IC_{50}$ > cutoff as 1; < cutoff as 0). Thus, low level resistance was treated differently than high resistance ($IC_{50}$ > max) in the MLR analysis. The actual $IC_{50}$ also allowed for assignment of a weight based on the coefficients derived from the MLR. In each patch, residues with significant correlation with neutralization response identified as p-value < 0.05, and/or SVM sensitivity or specificity > 80% (accuracy of SVM by 20-fold cross-validation > 70%) were stored as candidate epitopes. A weight was assigned to each candidate epitope, and the candidate epitope were scrutinized. Patches with agreement between multiple models were predicted with high likelihood to be potential epitopes. Figure 9 schematically illustrates a process of patch alignment. The sites, mutations which highly correlated with the changes in neutralization response, were identified by most significant coefficients/odds ratio or, for SVM, sensitivity/specificity ratio.

An example of a patch file is provided below:

| seqid | ic50_val | A501 | P498 | T499 | K500 |
|---|---|---|---|---|---|
| E05-112792-9 | 0.005 | 0 | 0 | 0 | 0 |
| E05-114413-11 | 50 | 0 | 0 | 0 | 0 |
| E05-114414-10 | 50 | 0 | 0 | 0 | 0 |
| E05-114414-16 | 50 | 0 | 0 | 0 | 0 |
| E05-114414-5 | 50 | 0 | 0 | 0 | 0 |
| E05-114415-14 | 50 | 0 | 0 | 0 | 0 |
| E05-114424-2 | 0.186 | 0 | 0 | 0 | 0 |

The column "seqid" contains identifiers assigned to each patch sequence variant. The column "ic50_val" contains $IC_{50}$ data (in units of concentration) for each sequence variant. This data is converted into binary form prior to analysis by computational methods.

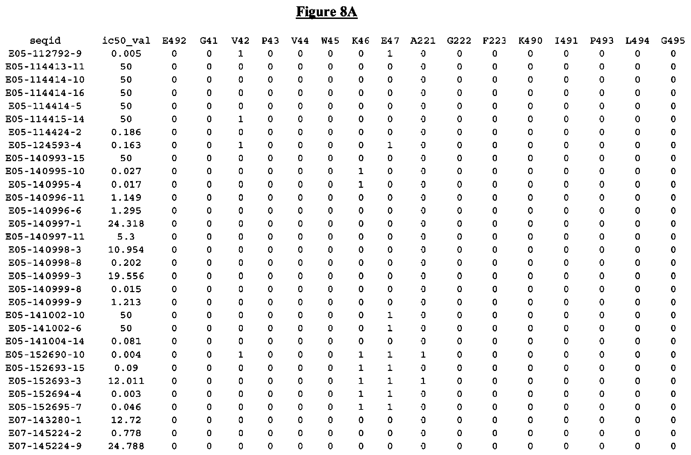

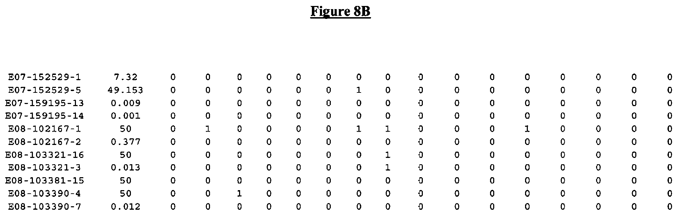

A501 - K500 are the identifiers for the amino acids residues within the patch. This patch example includes residues at positions 498, 499, 500, and 501. If a row with a particular sequence identifier has a 1 in a column for a particular residue, it means the sequence has a mutation, as compared to the wild-type sequence. Another example of a patch file is illustrated in Figures 8A and 8B.

The computational techniques used in the embodiments of the methods of the present invention can be implemented in any suitable computer language. In one example, the computational techniques were implemented in R. Some parameters used (as implemented in R) are shown below:

Multiple Linear Regression:

```
mylm = lm(ic50 ~ ., data=patch)
```

Logistic regression:

```
myLogr = glm(NeutResponse ~ ., data=patch,

family=binomial(link="logit") )
```

Fisher's Excat test:

fisher_data<- matrix(c(mut_resistant,mut_sensitive,noMut_resistant,noMut_sen sitive),nr=2,byrow=T); ftest <- c (fisher.test(fisher_data));

The SVM model was trained using libsvm in R package e1071 with the linear kernel, 20 cross-validation and default cost=1. The cutoff for SVM decision values was set at the default value of 0.

EXAMPLE 7

*B12, PG9 and PG16 Epitope identification*

PG9 and PG16 antibodies are known HIV-1-neutralizing antibodies that were originally isolated from an African clade A-infected donor and shown to be broad and potent. PG9 and PG16 are discussed, for example, in Doores and Burton "Variable Loop Glycan Dependency of the Broad and Potent HIV-1-Neutralizing Antibodies PG9 and PG16." Journal of Virology, 2010, 84:10510-10521; Walker et al. "Broad and potent neutralizing antibodies from an African donor reveal a new HIV-1 vaccine target." Science 2009, 326(5950):285-89. B12 is another known HIV-1 neutralizing antibody. B12, PG9, and PG16 epitopes are known to be located in gp120.

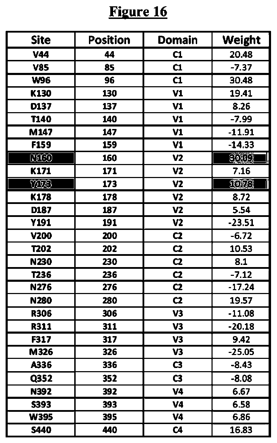

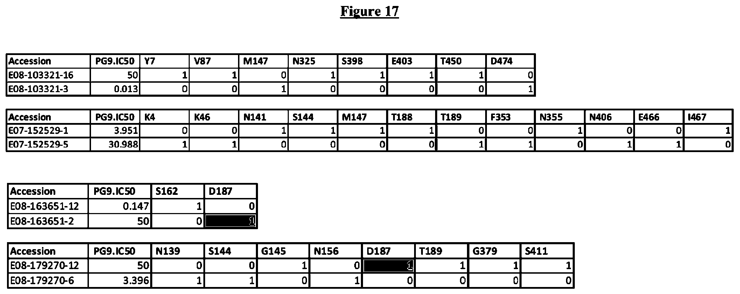

The results of the PG9 epitope identification by exemplary methods of epitope identification according to some embodiments of the present invention are schematically illustrated in Figures 10-17. The results of the PG16 epitope identification by embodiments of the methods of the present invention are shown in Figure 18 (also data not shown). The results of the b12 epitope identification by embodiments of the methods of the present invention are schematically illustrated in Figures 19 and 20. The results of the 2F5 epitope identification in gp41 by embodiments of the methods of the present invention are schematically illustrated in Figure 21. Figure 22 shows a comparison of PG9 and PG16 antibody epitopes identified by the embodiments of the present methods to previously reported epitopes (also data not shown). Most of the newly identified epitopes contained amino acids within the previously reported epitopes. The results of the epitope identification for PG9 and PG16 antibodies indicated that the methods of epitope identification according to some embodiments of the present methods provide an extremely useful and advantageous tool for rapid and scanning of protein structures and efficient identification of epitope regions. Embodiments of the present methods are also useful for identification of immunogenic regions, or immunological hotspots.

EXAMPLE 8

*2F5 Epitope identification*

The 2F5 antibody is a known HIV-1-neutralizing antibody. 2F5 epitopes are known to be located in gp41.
The results of the 2F5 epitope identification by exemplary methods of epitope identification according to some embodiments of the present invention are schematically illustrated in Figure 21. The results of the epitope identification for the 2F5 antibody demonstrated that the methods of epitope identification according to some embodiments of the present methods provide a useful and advantageous tool for rapid scanning of protein structures and identification of epitope regions. Embodiments of the present methods are also useful for identification of immunogenic regions, or immunological hotspots.

EXAMPLE 9

*Epitope identification for a group of broadly neutralizing HIV-1 antibodies*

Epitope identification for a group of broadly neutralizing HIV-1 antibodies was performed. The group of broadly neutralizing antibodies included B12, PG9, PG16, PGT121, PGT122, PGT123, PGT124, PGT125, PGT126, PGT127, PGT128, PGT130, PGT131, PGT135, PGT136, PGT137, PGT141, PGT142, PGT143, PGT144, PGT145, and PGV04 antibodies. The information on the neutralization titers ($IC_{50}$) for a set of 282 HIV sequences was obtained for each antibody. The patch files were generated according to the procedures discussed earlier. The patch identification process generated 268 unique patches on the gp120 surface. To assure the inclusion of residues that may be part of a RIN, patches with seven different radii (6, 8, 10, 12, 15, and 20 angstroms) and four different thresholds for maximum residue depth (0, 1.5, 2.5, and 3 angstroms) were generated for each patch center. The resulting patch files were used in subsequent steps.
The data mining methods were applied to all sets of patch vectors, each set corresponding to a particular antibody. Patches that included statistically significant (p-value < 0.05) amino acid sites by MLR, LR, or FET, or achieved an overall accuracy of > 70% by SVM cross-validation were marked as significant, and were considered for further evaluation as potential epitopes. No correction for multiple testing was performed in order to find the most potential antibody targets. The number of the computational methods that identified each patch as significant was noted, with the hypothesis that patches with highest agreement by multiple models were more likely to be substantially affecting the neutralization sensitivity, and therefore, more probable to be part of an epitope. In each significant patch, residues with significant correlation with the neutralization response identified as p-value < 0.05 were highlighted and stored as regions that are impactful with regards to the neutralization response to the antibody.

Within each patch condition, as defined by the particular patch radius and thickness, all patches that were determined to contain significant residues by all four of the data mining models were linearly aligned next to each other by residue position. To identify likely epitope residues, the frequency of occurrence of each residue across the significant patches was noted, and was normalized by the most frequently occurring residue to allow for consistent comparison across all patch sets for different antibodies. Amino acid positions that consistently appeared with a high normalized frequency ($\geq$ 50%) were determined. These residues were then examined across all different conditions. The residues occurring with a higher than average frequency (> 50%) within all the conditions explored, were considered to be epitope candidates for that particular antibody.

In order to visualize the spatial organization of the amino acids comprising a patch, candidate epitope residues were mapped onto the surface of the CPH gp120 protein monomer model and onto a theoretical gp120 trimer structure to provide a method for evaluating candidate epitopes arising from potential protein-protein interfaces. Predicted epitopes were then visually evaluated through comparison with known epitopes (data not shown), or superimposed upon other gp120 crystal structures such a 2NY7 which contained additional interacting molecules such as the b12 fab and CD4 to evaluate the accuracy of the epitope prediction.

gp120 amino acid residues with statistically significant correlation with $IC_{50}$ values were identified as residues that may impact neutralization sensitivity ("significant residues"). Significant residues were identified across different patch conditions, defined by patch atomic depth (dpx) and radius. The patches containing significant residues were aligned linearly, and the frequency of occurrence within the patches for each significant residue was determined. Patch residues with a normalized percent frequency >50% were considered potentially interacting residues. These potentially interacting residues were mapped onto a three-dimensional gp120 (data not shown), thus showing potential antibody binding sites.

Antibody epitopes were identified based on strong correlations of the sequence variations with the neutralization response to each broadly neutralizing antibody. The number of significant patches varied depending on the antibody under analysis. Some of the antibodies could be grouped into families as they originated from the same donor: PG-9 and PG-16, PGT-121-131, PGT-135-137, and PGT-141-145. The results of epitope identification for antibodies PG9 and PG16 are schematically illustrated in Figure 24. The epitopes of these families were spatially oriented into overlapping or neighboring clusters of residues, as shown in Figure 25. A number of the significant residues and of the patch clusters predicting neutralization response to the broadly neutralizing antibodies were shown to be located within V1/V2 and V3 regions of gp120 protein, as illustrated in Figure 25.

Identified epitopes for all the antibodies were mapped onto the theoretical full-length gp120 CPH model (data not shown). Notably, when the predicted epitopes for PG9 and PG16 were modeled on the surface of the CPH structure together with the published residues, a good overlap was observed (data not shown). This demonstrated that the improved patch analysis method can be utilized to identify protein regions that are most likely to be involved in antibody binding in order to identify candidates for potential vaccine immunogens.

To evaluate the performance of the epitope prediction method, SVM models using only the significant sites within gp120 as input were built. The overall accuracy of the prediction was examined by comparing the predicted neutralization sensitivity to the measured antibody titer, which was classified into positive or negative (IC50 $\geq$ or < max dilution, respectively). Sensitivity (true positive rate) and specificity (true negative rate) for detection of resistance to the particular antibody were calculated. In order to assure that the viral subtype does not impose a bias in terms of performance characteristics of the algorithm, the accuracy of the prediction was examined within each of the subtypes in which more than 10 samples existed. The standard-deviation was derived across each of these subtypes. When using the subset of residues identified by the algorithm as input into a predictive model, high concordance of the neutralization sensitivity with the predicted response (>80%) was achieved for all antibodies. This high concordance was maintained within all different antibody subtype groups, as demonstrated by the narrow range of standard-deviations (2.42 - 10.35), as illustrated in Figure 26, thus showing that antibody subtype was not a confounder with respect to the quality of epitope prediction.

Different arrangements and combinations of the elements and the features described herein are possible. Similarly, some features and subcombinations are useful and may be employed without reference to other features and subcombinations. Embodiments of the invention and examples have been described for illustrative and not restrictive purposes, and alternative embodiments will become apparent to readers of this patent.

**Claims**

1. A computer-implemented method for identifying a ligand binding site of a protein, comprising:

   a. obtaining sequence information for a plurality of sequence variants of the protein;
   b. obtaining a measure of the biological activity of each of the plurality of the sequence variants;
   c. using a three-dimensional model of the protein's structure to define patches on the protein structure containing a plurality of residues within close proximity of each other, wherein the residue at the center of the patch is determined based on solvent accessibility, and wherein the residues are within 20 angstroms of the residue at the center;
   d. correlating the sequence variability within each of the patches with the biological activity data for each sequence variant, wherein the information on the sequence variability within each patch and the biological activity data for each sequence variant is represented in a non-binary or binary form, compiled as a vector file, and processed by two or more computational methods testing each patch for an ability of the sequence variability within the patch to predict changes in the biological activity; wherein the two or more computational methods comprise i) at least one method involving multivariate analysis, at least one method involving univariate analysis, and at least one machine learning algorithm, or ii) two or more of multiple regression analysis, logistic regression analysis, support vector machine, Fischer's Exact test, Hidden Markov Models, Neural Networks, Random Forest, Decision Trees, or Bayesian Networks; and wherein a residue is identified as a potential binding site residue if variation at the residue exhibits statistically significant correlation with the biological activity;
   e. identifying the residues forming the binding site by comparing the potential binding site residues identified with the two or more computational methods and identifying residues based on the agreement between the two or more computational methods; and
   f. producing a vaccine or other immunogenic composition comprising an immunogen and a pharmaceutically acceptable carrier, wherein the immunogen comprises the binding site identified in step (e); or producing an antibody that interacts with the binding site identified in step (e), wherein the antibody is effective to disrupt the interaction of the protein with the ligand.

2. The method of Claim 1, wherein step (b) comprises performing experiments to determine the dissociation constant or the Michaelis constant or measuring the neutralizing activity of an antibody to the ligand on the sequence variant.

3. The method of any one of Claims 1 to 2, wherein the three-dimensional model is from a crystal structure or from a computer-generated model.

4. The method of any one of Claims 1 to 3, wherein the two or more computational methods comprise at least one method involving multivariate analysis, at least one method involving univariate analysis, and at least one machine learning algorithm.

5. The method of any one of Claims 1 to 4, wherein the data in step (d) is processed by two computational methods, three computational methods, or four or more computational methods.

6. The method of Claim 5, wherein the data in step (d) is processed using multiple linear regression, support vector machine, and Fischer's Exact test.

7. The method of Claim 5, wherein the data in step (d) is processed using multiple linear regression, logistic regression, support vector machine, and Fischer's Exact test.

8. A system for use with the method of claim 1 for identifying a ligand binding site of a protein, comprising:

   a computer readable medium; and
   a processor in communication with the computer readable medium, the program configured to:

   receive sequence information for a plurality of sequence variants of the protein;
   receive biological activity data of each of the plurality of the sequence variants for the ligand;
   receive data regarding a three-dimensional model of the protein's structure;
   apply two or more computational methods to the data;
   compare the results obtained by applying the computational methods; and
   identify at least one residue forming the binding site.

9. The system of Claim 8, wherein the two or more computational methods comprise a) at least one method involving multivariate analysis, at least one method involving univariate analysis, and at least one machine learning algorithm; or b) multiple regression analysis, logistic regression analysis, support vector machine, Fischer's Exact test, Hidden Markov Models, Neural Networks, Random Forest, Decision Trees, or Bayesian Networks, or a combination thereof.

10. A computer readable medium for use with the method of claim 1 comprising program code comprising:

> program code for receiving sequence data, the sequence data representing an amino acid sequence, nucleic acid sequence, or both of at least a portion of protein;
> program code for receiving biological activity data;
> program code for receiving data regarding a three-dimensional model of the protein's structure;
> program code for applying two or more computational methods to the data;
> program code for comparing the results obtained by applying the computational methods; and
> program code for identifying at least one residue forming the binding site.

## Patentansprüche

1. Computerimplementiertes Verfahren zum Identifizieren einer Ligandenbindungsstelle eines Proteins, umfassend:

> a. Erhalten von Sequenzinformationen für eine Vielzahl von Sequenzvarianten des Proteins;
> b. Erhalten eines Maßes für die biologische Aktivität jeder der Vielzahl von Sequenzvarianten;
> c. Verwenden eines dreidimensionalen Modells der Struktur des Proteins, um Bereiche auf der Proteinstruktur zu definieren, die eine Vielzahl von Resten in unmittelbarer Nähe zueinander enthalten, wobei der Rest in der Mitte des Bereichs basierend auf einer Lösungsmittelzugänglichkeit bestimmt wird und wobei die Reste innerhalb von 20 Angström um den Rest in der Mitte liegen;
> d. Korrelieren der Sequenzvariabilität innerhalb jedes Bereichs mit den Daten der biologischen Aktivität für jede Sequenzvariante, wobei die Informationen zu der Sequenzvariabilität innerhalb jedes Bereichs und die Daten der biologischen Aktivität für jede Sequenzvariante in einer nicht-binären oder binären Form dargestellt, als eine Vektordatei kompiliert und durch zwei oder mehr Rechenverfahren verarbeitet werden, die jeden Bereich auf eine Fähigkeit der Sequenzvariabilität innerhalb des Bereichs testen, um Änderungen der biologischen Aktivität vorherzusagen; wobei die zwei oder mehr Rechenverfahren i) mindestens ein Verfahren mit multivariater Analyse, mindestens ein Verfahren mit univariater Analyse und mindestens einen Algorithmus für maschinelles Lernen oder ii) zwei oder mehr umfassen von multipler Regressionsanalyse, logistischer Regressionsanalyse, Support-Vektor-Maschine, exaktem Test von Fischer, Hidden-Markov-Modellen, neuronalen Netzwerke, Random Forest, Entscheidungsbäumen oder Bayes-Netzwerken; und wobei ein Rest als ein potenzieller Bindungsstellenrest identifiziert wird, falls die Variation an dem Rest eine statistisch signifikante Korrelation mit der biologischen Aktivität vorweist;
> e. Identifizieren der Reste, die die Bindungsstelle ausbilden, durch ein Vergleichen der Reste der potenziellen Bindungsstelle, die mit den zwei oder mehr Rechenverfahren identifiziert werden, und Identifizieren der Reste basierend auf der Übereinstimmung zwischen den zwei oder mehr Rechenverfahren; und
> f. Herstellen eines Impfstoffs oder einer anderen immunogenen Zusammensetzung, umfassend ein Immunogen und einen pharmazeutisch verträglichen Träger, wobei das Immunogen die in Schritt (e) identifizierte Bindungsstelle umfasst; oder Herstellen eines Antikörpers, der mit der in Schritt (e) identifizierten Bindungsstelle interagiert, wobei der Antikörper wirksam ist, um die Interaktion des Proteins mit dem Liganden zu unterbrechen.

2. Verfahren nach Anspruch 1, wobei Schritt (b) das Durchführen von Experimenten, um die Dissoziationskonstante oder der Michaelis-Konstante zu bestimmen, oder das Messen der neutralisierenden Aktivität eines Antikörpers gegen den Liganden auf der Sequenzvariante umfasst.

3. Verfahren nach einem der Ansprüche 1 bis 2, wobei das dreidimensionale Modell aus einer Kristallstruktur oder einem computergenerierten Modell stammt.

4. Verfahren nach einem der Ansprüche 1 bis 3, wobei die zwei oder mehr Rechenverfahren mindestens ein Verfahren mit multivariater Analyse, mindestens ein Verfahren mit univariater Analyse und mindestens einen Algorithmus für maschinelles Lernen umfassen.

5. Verfahren nach einem der Ansprüche 1 bis 4, wobei die Daten in Schritt (d) durch zwei Rechenverfahren, drei Rechenverfahren oder vier oder mehr Rechenverfahren verarbeitet werden.

6. Verfahren nach Anspruch 5, wobei die Daten in Schritt (d) unter Verwendung von multipler linearer Regression, einer Support-Vektor-Maschine und des exakten Tests von Fischer verarbeitet werden.

7. Verfahren nach Anspruch 5, wobei die Daten in Schritt (d) unter Verwendung von multipler linearer Regression, logistischer Regression, einer Support-Vektor-Maschine und des exakten Tests von Fischer verarbeitet werden.

8. System zur Verwendung mit dem Verfahren nach Anspruch 1 zum Identifizieren einer Ligandenbindungsstelle eines Proteins, umfassend:

   ein computerlesbares Medium; und
   einen Prozessor in Kommunikation mit dem computerlesbaren Medium, wobei das Programm konfiguriert ist zum:

   Empfangen von Sequenzinformationen für eine Vielzahl von Sequenzvarianten des Proteins;
   Empfangen von Daten der biologischen Aktivität jeder der Vielzahl von Sequenzvarianten für den Liganden;
   Empfangen von Daten zu einem dreidimensionalen Modell der Proteinstruktur;
   Anwenden von zwei oder mehr Rechenverfahren auf die Daten;
   Vergleichen der Ergebnisse, die durch das Anwenden der Rechenverfahren erhalten werden; und
   Identifizieren mindestens eines Rests, der die Bindungsstelle ausbildet.

9. System nach Anspruch 8, wobei die zwei oder mehr Rechenverfahren a) mindestens ein Verfahren mit multivariater Analyse, mindestens ein Verfahren mit univariater Analyse und mindestens einen Algorithmus für maschinelles Lernen; oder b) multiple Regressionsanalyse, logistische Regressionsanalyse, Support-Vektor-Maschine, exakten Test von Fischer, Hidden-Markov-Modelle, neuronale Netzwerke, Random Forest, Entscheidungsbäume oder Bayes-Netzwerke oder eine Kombination davon umfassen.

10. Computerlesbares Medium zur Verwendung mit dem Verfahren nach Anspruch 1, umfassend einen Programmcode, umfassend:

   Programmcode zum Empfangen von Sequenzdaten, wobei die Sequenzdaten eine Aminosäuresequenz, eine Nukleinsäuresequenz oder beides von mindestens einem Abschnitt eines Proteins darstellen;
   Programmcode zum Empfangen biologischer Aktivitätsdaten;
   Programmcode zum Empfangen von Daten bezüglich eines dreidimensionalen Modells der Struktur des Proteins;
   Programmcode zum Anwenden von zwei oder mehr Rechenverfahren auf die Daten;
   Programmcode zum Vergleichen der Ergebnisse, die durch das Anwenden der Rechenverfahren erhalten werden; und
   Programmcode zum Identifizieren mindestens eines Rests, der die Bindungsstelle ausbildet.

**Revendications**

1. Procédé implémenté par ordinateur permettant d'identifier un site de liaison de ligand d'une protéine, comprenant :

   a. l'obtention d'informations de séquence pour une pluralité de variants de séquence de la protéine ;
   b. l'obtention d'une mesure de l'activité biologique de chacun parmi la pluralité des variants de séquence ;
   c. l'utilisation d'un modèle tridimensionnel de la structure de la protéine pour définir des patchs sur la structure protéique contenant une pluralité de résidus très proches les uns des autres, dans lequel le résidu au centre du patch est déterminé en fonction d'une accessibilité de solvant, et dans lequel les résidus sont à moins de 20 angströms du résidu au centre ;
   d. la corrélation de la variabilité de séquence au sein de chacun des patchs avec les données d'activité biologique pour chaque variant de séquence, dans lequel les informations relatives à la variabilité de séquence au sein de chaque patch et les données d'activité biologique pour chaque variant de séquence sont représentées sous une forme non binaire ou binaire, compilées en tant que fichier vectoriel, et traitées par deux procédés de calcul ou plus testant chaque patch pour déceler une aptitude de la variabilité de séquence au sein du patch à prédire des changements dans l'activité biologique ; dans lequel les deux procédés de calcul ou plus comprennent i) au moins un procédé impliquant une analyse à variables multiples, au moins un procédé impliquant une analyse à une seule variable, et au moins un algorithme d'apprentissage automatique, ou ii) deux ou plus parmi une analyse de régression multiple, une analyse de régression logistique, une machine à vecteurs de support, un test exact de Fischer, des modèles de Markov cachés, des réseaux neuronaux, une arborescence aléatoire, des schémas décisionnels ou des réseaux bayésiens ; et dans lequel un résidu est identifié en tant que résidu de site de liaison potentiel si une variation au niveau du résidu présente une corrélation statistiquement significative avec l'activité biologique ;

e. l'identification des résidus formant le site de liaison en comparant les résidus de site de liaison potentiel identifiés avec les deux procédés de calcul ou plus, et en identifiant des résidus en fonction de la concordance entre les deux procédés de calcul ou plus ; et

f. la production d'un vaccin ou d'une autre composition immunogène comprenant un immunogène et un support pharmaceutiquement acceptable, dans lequel l'immunogène comprend le site de liaison identifié à l'étape (e) ; ou la production d'un anticorps qui interagit avec le site de liaison identifié à l'étape (e), dans lequel l'anticorps est efficace pour perturber l'interaction de la protéine avec le ligand.

2. Procédé selon la revendication 1, dans lequel l'étape (b) comprend la mise en œuvre d'expériences pour déterminer la constante de dissociation ou la constante de Michaelis ou la mesure de l'activité neutralisante d'un anticorps pour le ligand sur le variant de séquence.

3. Procédé selon l'une quelconque des revendications 1 à 2, dans lequel le modèle tridimensionnel provient d'une structure cristalline ou d'un modèle généré par ordinateur.

4. Procédé selon l'une quelconque des revendications 1 à 3, dans lequel les deux procédés de calcul ou plus comprennent au moins un procédé impliquant une analyse à variables multiples, au moins un procédé impliquant une analyse à une seule variable, et au moins un algorithme d'apprentissage automatique.

5. Procédé selon l'une quelconque des revendications 1 à 4, dans lequel les données à l'étape (d) sont traitées par deux procédés de calcul, trois procédés de calcul, ou quatre procédés de calcul ou plus.

6. Procédé selon la revendication 5, dans lequel les données à l'étape (d) sont traitées à l'aide d'une régression linéaire multiple, d'une machine à vecteurs de support, et d'un test exact de Fischer.

7. Procédé selon la revendication 5, dans lequel les données à l'étape (d) sont traitées à l'aide d'une régression linéaire multiple, d'une régression logistique, d'une machine à vecteurs de support, et d'un test exact de Fischer.

8. Système pour utilisation avec le procédé selon la revendication 1 permettant d'identifier un site de liaison de ligand d'une protéine, comprenant :

un support lisible par ordinateur ; et
un processeur en communication avec le support lisible par ordinateur, le programme étant configuré pour :

recevoir des informations de séquence pour une pluralité de variants de séquence de la protéine ;
recevoir des données d'activité biologique de chacun parmi la pluralité des variants de séquence pour le ligand ;
recevoir des données concernant un modèle tridimensionnel de la structure de la protéine ;
appliquer deux procédés de calcul ou plus aux données ;
comparer les résultats obtenus en appliquant les procédés de calcul ; et
identifier au moins un résidu formant le site de liaison.

9. Système selon la revendication 8, dans lequel les deux procédés de calcul ou plus comprennent a) au moins un procédé impliquant une analyse à variables multiples, au moins un procédé impliquant une analyse à une seule variable, et au moins un algorithme d'apprentissage automatique ; ou b) une analyse de régression multiple, une analyse de régression logistique, une machine à vecteurs de support, un test exact de Fischer, des modèles de Markov cachés, des réseaux neuronaux, une arborescence aléatoire, des schémas décisionnels, ou des réseaux bayésiens, ou une combinaison de ceux-ci.

10. Support lisible par ordinateur pour utilisation avec le procédé selon la revendication 1 comprenant un code de programme comprenant :

un code de programme permettant de recevoir des données de séquence, les données de séquence représentant une séquence d'acides aminés, une séquence d'acides nucléiques, ou l'une et l'autre d'au moins une partie de protéine ;

un code de programme permettant de recevoir des données d'activité biologique ;

un code de programme permettant de recevoir des données concernant un modèle tridimensionnel de la structure de la protéine ;

un code de programme permettant d'appliquer deux procédés de calcul ou plus aux données ;

un code de programme permettant de comparer les résultats obtenus en appliquant les procédés de calcul ; et

un code de programme permettant d'identifier au moins un résidu formant le site de liaison.

Sequence information for sequence variants

Reference sequence variant

Sequence alignment

Experimental Data

Create Patch Lists

Create patch vector files:
- Identify mutations and convert each patch sequence variant into 0/1 vector file based on the mutations present in the variant
- Convert experimental data for each sequence variant into a binary form and create a suitable vector
- Merge sequence vectors and affinity vectors

3-D Protein Model

Visualize and examine most likely patches on the 3-D Protein model

Patch analysis:
- Evaluate each patch vector file
  - Multiple Linear Regression
  - SVM
  - Logistic Regression
  - Fisher exact test
- Report significant patches for each evaluation

**Figure 1A**

- Compare results obtained from different evaluations
- Determine most likely patches based on the comparison

Figure 1B

Figure 2

Figure 3

Figure 4

Figure 5

Figure 6

Figure 7

**Figure 8A**

| seqid | ic50_val | E492 | G41 | V42 | P43 | V44 | W45 | K46 | E47 | A221 | G222 | F223 | K490 | I491 | P493 | L494 | G495 |
|---|---|---|---|---|---|---|---|---|---|---|---|---|---|---|---|---|---|
| E05-112792-9 | 0.005 | 0 | 0 | 1 | 0 | 0 | 0 | 0 | 1 | 0 | 0 | 0 | 0 | 0 | 0 | 0 | 0 |
| E05-114413-11 | 50 | 0 | 0 | 0 | 0 | 0 | 0 | 0 | 0 | 0 | 0 | 0 | 0 | 0 | 0 | 0 | 0 |
| E05-114414-10 | 50 | 0 | 0 | 0 | 0 | 0 | 0 | 0 | 0 | 0 | 0 | 0 | 0 | 0 | 0 | 0 | 0 |
| E05-114414-16 | 50 | 0 | 0 | 0 | 0 | 0 | 0 | 0 | 0 | 0 | 0 | 0 | 0 | 0 | 0 | 0 | 0 |
| E05-114414-5 | 50 | 0 | 0 | 0 | 0 | 0 | 0 | 0 | 0 | 0 | 0 | 0 | 0 | 0 | 0 | 0 | 0 |
| E05-114415-14 | 50 | 0 | 0 | 1 | 0 | 0 | 0 | 0 | 0 | 0 | 0 | 0 | 0 | 0 | 0 | 0 | 0 |
| E05-114424-2 | 0.186 | 0 | 0 | 0 | 0 | 0 | 0 | 0 | 0 | 0 | 0 | 0 | 0 | 0 | 0 | 0 | 0 |
| E05-124593-4 | 0.163 | 0 | 0 | 1 | 0 | 0 | 0 | 0 | 1 | 0 | 0 | 0 | 0 | 0 | 0 | 0 | 0 |
| E05-140993-15 | 50 | 0 | 0 | 0 | 0 | 0 | 0 | 0 | 0 | 0 | 0 | 0 | 0 | 0 | 0 | 0 | 0 |
| E05-140995-10 | 0.027 | 0 | 0 | 0 | 0 | 0 | 0 | 1 | 0 | 0 | 0 | 0 | 0 | 0 | 0 | 0 | 0 |
| E05-140995-4 | 0.017 | 0 | 0 | 0 | 0 | 0 | 0 | 1 | 0 | 0 | 0 | 0 | 0 | 0 | 0 | 0 | 0 |
| E05-140996-11 | 1.149 | 0 | 0 | 0 | 0 | 0 | 0 | 0 | 0 | 0 | 0 | 0 | 0 | 0 | 0 | 0 | 0 |
| E05-140996-6 | 1.295 | 0 | 0 | 0 | 0 | 0 | 0 | 0 | 0 | 0 | 0 | 0 | 0 | 0 | 0 | 0 | 0 |
| E05-140997-1 | 24.318 | 0 | 0 | 0 | 0 | 0 | 0 | 0 | 0 | 0 | 0 | 0 | 0 | 0 | 0 | 0 | 0 |
| E05-140997-11 | 5.3 | 0 | 0 | 0 | 0 | 0 | 0 | 0 | 0 | 0 | 0 | 0 | 0 | 0 | 0 | 0 | 0 |
| E05-140998-3 | 10.954 | 0 | 0 | 0 | 0 | 0 | 0 | 0 | 0 | 0 | 0 | 0 | 0 | 0 | 0 | 0 | 0 |
| E05-140998-8 | 0.202 | 0 | 0 | 0 | 0 | 0 | 0 | 0 | 0 | 0 | 0 | 0 | 0 | 0 | 0 | 0 | 0 |
| E05-140999-3 | 19.556 | 0 | 0 | 0 | 0 | 0 | 0 | 0 | 0 | 0 | 0 | 0 | 0 | 0 | 0 | 0 | 0 |
| E05-140999-8 | 0.015 | 0 | 0 | 0 | 0 | 0 | 0 | 0 | 0 | 0 | 0 | 0 | 0 | 0 | 0 | 0 | 0 |
| E05-140999-9 | 1.213 | 0 | 0 | 0 | 0 | 0 | 0 | 0 | 0 | 0 | 0 | 0 | 0 | 0 | 0 | 0 | 0 |
| E05-141002-10 | 50 | 0 | 0 | 0 | 0 | 0 | 0 | 0 | 1 | 0 | 0 | 0 | 0 | 0 | 0 | 0 | 0 |
| E05-141002-6 | 50 | 0 | 0 | 0 | 0 | 0 | 0 | 0 | 1 | 0 | 0 | 0 | 0 | 0 | 0 | 0 | 0 |
| E05-141004-14 | 0.081 | 0 | 0 | 0 | 0 | 0 | 0 | 0 | 0 | 0 | 0 | 0 | 0 | 0 | 0 | 0 | 0 |
| E05-152690-10 | 0.004 | 0 | 0 | 1 | 0 | 0 | 0 | 1 | 1 | 1 | 0 | 0 | 0 | 0 | 0 | 0 | 0 |
| E05-152693-15 | 0.09 | 0 | 0 | 0 | 0 | 0 | 0 | 1 | 1 | 1 | 0 | 0 | 0 | 0 | 0 | 0 | 0 |
| E05-152693-3 | 12.011 | 0 | 0 | 0 | 0 | 0 | 0 | 1 | 1 | 1 | 0 | 0 | 0 | 0 | 0 | 0 | 0 |
| E05-152694-4 | 0.003 | 0 | 0 | 0 | 0 | 0 | 0 | 1 | 1 | 0 | 0 | 0 | 0 | 0 | 0 | 0 | 0 |
| E05-152695-7 | 0.046 | 0 | 0 | 0 | 0 | 0 | 0 | 1 | 1 | 0 | 0 | 0 | 0 | 0 | 0 | 0 | 0 |
| E07-143280-1 | 12.72 | 0 | 0 | 0 | 0 | 0 | 0 | 0 | 0 | 0 | 0 | 0 | 0 | 0 | 0 | 0 | 0 |
| E07-145224-2 | 0.778 | 0 | 0 | 0 | 0 | 0 | 0 | 0 | 0 | 0 | 0 | 0 | 0 | 0 | 0 | 0 | 0 |
| E07-145224-9 | 24.788 | 0 | 0 | 0 | 0 | 0 | 0 | 0 | 0 | 0 | 0 | 0 | 0 | 0 | 0 | 0 | 0 |

EP 4 050 609 B1

**Figure 8B**

| E07-152529-1  | 7.32   | 0 | 0 | 0 | 0 | 0 | 0 | 0 | 0 | 0 | 0 | 0 | 0 | 0 | 0 | 0 | 0 |
| E07-152529-5  | 49.153 | 0 | 0 | 0 | 0 | 0 | 0 | 1 | 0 | 0 | 0 | 0 | 0 | 0 | 0 | 0 | 0 |
| E07-159195-13 | 0.009  | 0 | 0 | 0 | 0 | 0 | 0 | 0 | 0 | 0 | 0 | 0 | 0 | 0 | 0 | 0 | 0 |
| E07-159195-14 | 0.001  | 0 | 0 | 0 | 0 | 0 | 0 | 0 | 0 | 0 | 0 | 0 | 0 | 0 | 0 | 0 | 0 |
| E08-102167-1  | 50     | 0 | 1 | 0 | 0 | 0 | 0 | 1 | 1 | 0 | 0 | 0 | 1 | 0 | 0 | 0 | 0 |
| E08-102167-2  | 0.377  | 0 | 0 | 0 | 0 | 0 | 0 | 0 | 0 | 0 | 0 | 0 | 0 | 0 | 0 | 0 | 0 |
| E08-103321-16 | 50     | 0 | 0 | 0 | 0 | 0 | 0 | 0 | 1 | 0 | 0 | 0 | 0 | 0 | 0 | 0 | 0 |
| E08-103321-3  | 0.013  | 0 | 0 | 0 | 0 | 0 | 0 | 0 | 1 | 0 | 0 | 0 | 0 | 0 | 0 | 0 | 0 |
| E08-103381-15 | 50     | 0 | 0 | 0 | 0 | 0 | 0 | 0 | 0 | 0 | 0 | 0 | 0 | 0 | 0 | 0 | 0 |
| E08-103390-4  | 50     | 0 | 0 | 1 | 0 | 0 | 0 | 0 | 0 | 0 | 0 | 0 | 0 | 0 | 0 | 0 | 0 |
| E08-103390-7  | 0.012  | 0 | 0 | 0 | 0 | 0 | 0 | 0 | 0 | 0 | 0 | 0 | 0 | 0 | 0 | 0 | 0 |

**Figure 9**

Patch 1
Patch 2
Patch 3
Patch 4
Patch 5

Primary epitope

Secondary epitope

## Figure 10A

```
D113 Q114 K117 C119 V120 L122                                                                                          S162
D113 Q114 K117 C119 V120
          K117 C119 V120
               C119
               C119 V120
                         L122 P124
                              C131 T132 T138      G145 R146 M147 I148 M149 E150
                                                                   I148 M149 E150 G152 I154 K155 C157 S158 F159 N160 S162
                                                                        M149 E150 G152 I154 K155 C157 S158 F159 N160 S162
                                                                             G152 I154 K155 C157 S158           S162
                                                                                  I154 K155 C157 S158      N160 S162
                                                                                  I154 K155 C157 S158           S162
                                                                                       K155 C157 S158 F159 N160 S162
                                                                                       K155 C157 S158      N160 S162
                                                                                       K155 C157 S158           S162
                                                                                            C157 S158           S162
                                                                                                 S158           S162
                                                                                                 S158           S162
                                                                                                                S162
```

```
 2    2    3    5    4    2    1    1    1    1    1    1    1    2    3    3    3    5    8    9   12    3    5   14
```

```
25 ┤
20 ┤
15 ┤
10 ┤
 5 ┤
 0 ┤
    D113 Q114 K117 C119 V120 L122 P124 C131 T132 T138 G145 R146 M147 I148 M149 E150 G152 I154 K155 C157 S158 F159 N160 S162
```

**Figure 10B**

S162  I165  R166  G167  K168  V169  Q170  K171  E172  Y173  A174  F175  F176  Y177  K178  L179  D180  I181  D185  D187  T188

Figure 10C

**Figure 11**

K305
I307
I181
I173

Primary epitope residues
Secondary epitope residues
Published epitope residues

Figure 12

Primary epitope residues
Secondary epitope residues
Published epitope residues

F176
F159
S158
S162
N160
I307
I181

Figure 13

I307

I423

I181

K305

Primary epitope residues
Secondary epitope residues
Published epitope residues

EP 4 050 609 B1

Figure 14

Primary epitope residues
Secondary epitope residues
Published epitope residues

Figure 15

| PG9 - GP120 *(CPH model)* | | | | |
|---|---|---|---|---|
| Patch | Domain | #Models | Significant Site | Weight |
| **2.5 Depth - 12 Ang** | | | | |
| ...N160-I165-R166-G167-K168-V169-Q170-K171-F175-F176-K178... | V2 | 4 | N160 | 30.09 |
| | V2 | 4 | K171 | 7.16 |
| | V2 | 4 | K178 | 8.72 |
| ...Y173-A174-F175-F176-Y177-T188-T189-S190-Y191-K192-L193-T194... | V2 | 4 | Y173 | 10.78 |
| ...D187-T202-A204... | C2 | 4 | T202 | 10.53 |
| | V2 | 4 | D187 | 5.54 |
| ...W96-K97-D99-N234-T236... | C1 | 4 | W96 | 30.48 |
| | C2 | 4 | T236 | -7.12 |
| ...K130-C131-D137-T138-N139-T140-N141-S142-S144-R146-M147-I148-M149-G152-C157- | V1 | 4 | K130 | 19.41 |
| | V1 | 4 | D137 | 8.26 |
| | V1 | 4 | T140 | -7.99 |
| | V1 | 4 | M147 | -11.91 |
| | C1 | 4 | F159 | -14.33 |
| ...T63-V68-K117-P206-V208-E211-G379-P438-I439-S440... | C4 | 4 | S440 | 16.83 |
| W96-N234-T236-V275-N276-F277-T278-D279-N280 | C2 | 4 | N276 | -17.24 |
| | C2 | 4 | N280 | 19.57 |
| K171-E172-Y173-A174-F175-F176-Y177-K178-D185-D187-T189-S190-Y191-C196-N197-V2( | V2 | 4 | Y191 | -23.51 |
| | C2 | 4 | V200 | -6.72 |
| ...R306-I307-I309-Q310-R311-G312-F317 | V3 | 3 | R306 | -11.08 |
| | V3 | 3 | R311 | -20.18 |
| | V3 | 3 | F317 | 9.42 |
| V318-T319-I320-G321-K322-I323-G324-N325-M326-R327-R335-A336... | V3 | 3 | M326 | -25.05 |
| ...A336-K337-N339-N340-K343-Q389-N392-S393... | C3 | 3 | A336 | -8.43 |
| | V4 | 3 | N392 | 6.67 |
| | V4 | 3 | S393 | 6.58 |
| ...V44-W45-V84-V85-V87-N88-T90-N92-N230-P238... | C1 | 3 | V44 | 20.48 |
| | C1 | 3 | V85 | -7.37 |
| | C2 | 3 | N230 | 8.1 |
| ...Q352-G354-N356-W395... | C3 | 3 | Q352 | -8.08 |
| | V4 | 3 | W395 | 6.86 |

## Figure 16

| Site | Position | Domain | Weight |
|------|----------|--------|--------|
| V44 | 44 | C1 | 20.48 |
| V85 | 85 | C1 | -7.37 |
| W96 | 96 | C1 | 30.48 |
| K130 | 130 | V1 | 19.41 |
| D137 | 137 | V1 | 8.26 |
| T140 | 140 | V1 | -7.99 |
| M147 | 147 | V1 | -11.91 |
| F159 | 159 | V1 | -14.33 |
| N160 | 160 | V2 | 30.09 |
| K171 | 171 | V2 | 7.16 |
| Y173 | 173 | V2 | 19.73 |
| K178 | 178 | V2 | 8.72 |
| D187 | 187 | V2 | 5.54 |
| Y191 | 191 | V2 | -23.51 |
| V200 | 200 | C2 | -6.72 |
| T202 | 202 | C2 | 10.53 |
| N230 | 230 | C2 | 8.1 |
| T236 | 236 | C2 | -7.12 |
| N276 | 276 | C2 | -17.24 |
| N280 | 280 | C2 | 19.57 |
| R306 | 306 | V3 | -11.08 |
| R311 | 311 | V3 | -20.18 |
| F317 | 317 | V3 | 9.42 |
| M326 | 326 | V3 | -25.05 |
| A336 | 336 | C3 | -8.43 |
| Q352 | 352 | C3 | -8.08 |
| N392 | 392 | V4 | 6.67 |
| S393 | 393 | V4 | 6.58 |
| W395 | 395 | V4 | 6.86 |
| S440 | 440 | C4 | 16.83 |

**Figure 17**

| Accession | PG9.IC50 | Y7 | V87 | M147 | N325 | S398 | E403 | T450 | D474 |
|---|---|---|---|---|---|---|---|---|---|
| E08-103321-16 | 50 | 1 | 1 | 1 | 0 | 1 | 1 | 1 | 0 |
| E08-103321-3 | 0.013 | 0 | 0 | 1 | 1 | 0 | 0 | 0 | 1 |

| Accession | PG9.IC50 | K4 | K46 | N141 | S144 | M147 | T188 | T189 | F353 | N355 | N406 | E466 | I467 |
|---|---|---|---|---|---|---|---|---|---|---|---|---|---|
| E07-152529-1 | 3.951 | 0 | 0 | 1 | 1 | 1 | 1 | 1 | 0 | 0 | 1 | 0 | 1 |
| E07-152529-5 | 30.988 | 1 | 1 | 0 | 0 | 0 | 0 | 1 | 1 | 0 | 0 | 1 | 0 |

| Accession | PG9.IC50 | S162 | D187 |
|---|---|---|---|
| E08-163651-12 | 0.147 | 1 | 0 |
| E08-163651-2 | 50 | 0 | 1 |

| Accession | PG9.IC50 | N139 | S144 | G145 | N156 | D187 | T189 | G379 | S411 |
|---|---|---|---|---|---|---|---|---|---|
| E08-179270-12 | 50 | 0 | 0 | 0 | 1 | 0 | 1 | 1 | 1 |
| E08-179270-6 | 3.396 | 1 | 1 | 1 | 0 | 1 | 0 | 0 | 0 |

**Figure 18**

| Site | Position | Domain | Weight |
|------|----------|--------|--------|
| W96 | 96 | C1 | 32.04 |
| L122 | 122 | C1/V1 | 29.1 |
| G145 | 145 | V1 | 9.88 |
| M147 | 147 | V1 | -12.22 |
| N160 | 160 | V2 | 27.44 |
| K171 | 171 | V2 | 8.33 |
| Y173 | 173 | V2 | 7.8 |
| K178 | 178 | V2 | 7.7 |
| D187 | 187 | V2 | 6.91 |
| V200 | 200 | C2 | -9.86 |
| T202 | 202 | C2 | 7.92 |
| T236 | 236 | C2 | -8.15 |
| N276 | 276 | C2 | -20.3 |
| F277 | 277 | C2 | 10.08 |
| N280 | 280 | C2 | 25.47 |
| T303 | 303 | V3 | 11.61 |
| R311 | 311 | V3 | -16.47 |
| F317 | 317 | V3 | 14.18 |
| M326 | 326 | V3 | -32.19 |
| K337 | 337 | C3 | 9.18 |
| G354 | 354 | C3 | -6.87 |
| N392 | 392 | V4 | 10.12 |
| W395 | 395 | V4 | 8 |
| Q442 | 442 | C4 | 11.38 |
| T455 | 455 | C4 | 10.23 |

EP 4 050 609 B1

**Figure 19**

| Site | Position | Domain |
|------|----------|--------|
| V84 | 84 | C1 |
| W96 | 96 | C1 |
| E102 | 102 | C1 |
| Q114 | 114 | C1 |
| I148 | 148 | V1 |
| E150 | 150 | V1 |
| V169 | 169 | V2 |
| Q170 | 170 | V2 |
| D185 | 185 | V2 |
| V200 | 200 | C2 |
| T236 | 236 | C2 |
| T240 | 240 | C2 |
| R252 | 252 | C2 |
| F277 | 277 | C2 |
| R315 | 315 | V3 |
| V318 | 318 | V3 |
| G354 | 354 | C3 |
| N355 | 355 | C3 |
| V372 | 372 | C3 |
| Q389 | 389 | V4 |
| F396 | 396 | V4 |
| K432 | 432 | C4 |
| Q442 | 442 | C4 |

■ Associated with reduced susceptibility with p-value <0.0001

☐ Associated with reduced susceptibility with p-value <0.05

☐ Associated with increased susceptibility with p-value <0.05

Figure 20

gp120

V-regions

Gp120
contact
residues

Predicted
primary
epitope

**Figure 21**

| Patch | Site | Position |
|---|---|---|
| K1-R2-A3-V4-G5 | K1 | 1 |
| G5-I6-G7-A8-L9 | I6 | 6 |
| T97-A98-V99-P100-W101 | P100 | 100 |
| T117-T118-W119-M120-E121 | T117 | 117 |
| W114-N115-H116-T117-T118 | T118 | 118 |
| M120-E121-W122-D123-R124 | R124 | 124 |
| E153-L154-D155-K156-W157 * | K156 | 156 |
| A158-S159-L160-W161-N162 * | A158 | 158 |
| N168-W169-L170-W171-Y172 | W171 | 171 |

■ Associated with reduced susceptibility with p-value <0.0001

☐ Associated with reduced susceptibility with p-value <0.05

☐ Associated with <u>increased</u> susceptibility with p-value <0.05

*: known epitope

Figure 22

## Figure 23

**Model Data**

3D-Model

Web

RSA          DPX

Create Patch Lists

Patch width (Å)
6    8    10   12   15   20

Patch Combinations

**Assay Data**

mAbs

IC50

Viral DNA sequences ◄─── Viruses

IC50 mAb

Create Patch Vector Files

- Identify mutations in each seq
- Merge IC50 data with mutations found in a patch as a vector
- Do for every mAb, patch list combination

Result Evaluation

- Identify patches containing significant residues
- Align significant patches
- Determine patch residue frequency
- Identify likely epitope residues

Patch Analysis

- Evaluate every patch vector file using multiple statistical models
- Report out significant patches for each mAb / patch list combination in each model

- Map putative epitopes to model for each condition tested

52

**Figure 24**

| PG9 | PG16 | Domain |
|---|---|---|
| Y39 | | C1 |
| V44 | | C1 |
| | T63 | C1 |
| | V68 | C1 |
| V85 | | C1 |
| W96 | W96 | C1 |
| | D99 | C1 |
| | L122 | C1 |
| K130 | | C1 |
| D137 | | V1 |
| T140 | | V1 |
| | N141 | V1 |
| G145 | G145 | V1 |
| M147 | M147 | V1 |
| | S158 | V2 |
| F159 | | V2 |
| N160 ** | N160 ** | V2 |
| | I165 | V2 |
| R166 | R166 | V2 |
| | V169 | V2 |
| | Q170 | V2 |
| K171 | K171 | V2 |
| Y173 ** | Y173 | V2 |
| A174 | | V2 |
| F176 | | V2 |
| K178 | K178 | V2 |
| | D187 | V2 |
| Y191 | | V2 |
| T194 | | V2 |
| V200 | V200 | C2 |
| T202 | T202 | C2 |
| A204 | A204 | C2 |
| N230 | | C2 |
| T236 | T236 | C2 |
| N276 | N276 | C2 |
| | F277 | C2 |
| N280 | N280 | C2 |
| | N289 | C2 |
| R311 | R311 | V3 |
| F317 | F317 ** | V3 |
| | T319 | V3 |
| M326 | M326 | V3 |
| A336 | | C3 |
| | K337 | C3 |
| Q344 | | C3 |
| Q352 | Q352 | C3 |
| | G354 | C3 |
| | P369 | C3 |
| N392 | N392 | V4 |
| W395 | W395 | V4 |
| | N397 | V4 |
| | S398 | V4 |
| W400 | W400 | V4 |
| K432 | K432 | C4 |
| | P437 | C4 |
| S440 | S440 | C4 |
| E492 | Q442 | C4 |
| | T455 | C4 |
| | N460 | V5 |
| | D474 | C5 |
| | R476 | C5 |
| | K490 | C5 |

EP 4 050 609 B1

Figure 25A

Figure 25B

# Figure 26

| Antibody | # Responders | # NonResponders | Sensitivity (%) | Specificity (%) | Accuracy (%) | A (N=21) | AE (N=24) | AG (N=19) | B (N=55) | C (N=32) | D (N=55) | F1 (N=24) | G (N=41) | Standard-Deviation Across Subtypes |
|---|---|---|---|---|---|---|---|---|---|---|---|---|---|---|
| | | | | | Algorithm Performance Characteristics | | | | % Accuracy within Subtypes | | | | | |
| B12 | 61 | 221 | 98.2 | 77 | 93.6 | 90.5 | 91.7 | 89.5 | 92.7 | 96.9 | 94.5 | 100 | 92.7 | 3.47 |
| PG16 | 177 | 105 | 74.3 | 93.2 | 86.2 | 90.5 | 83.3 | 94.7 | 89.1 | 75 | 89.1 | 83.3 | 82.9 | 6.10 |
| PG9 | 178 | 104 | 64.4 | 94.4 | 83.3 | 85.7 | 91.7 | 94.7 | 87.3 | 71.9 | 81.8 | 83.3 | 78.6 | 7.65 |
| PGT-121 | 165 | 117 | 88 | 97.6 | 93.6 | 100 | 100 | 100 | 90.9 | 90.6 | 90.9 | 91.7 | 95.1 | 4.45 |
| PGT-122 | 150 | 132 | 88.6 | 92 | 90.4 | 95.2 | 100 | 89.5 | 90.9 | 90.6 | 85.5 | 83.3 | 92.7 | 5.26 |
| PGT-123 | 159 | 123 | 82.9 | 93.1 | 88.7 | 85.7 | 100 | 84.2 | 89.1 | 93.8 | 87.3 | 83.3 | 87.8 | 5.54 |
| PGT-125 | 130 | 152 | 90.8 | 82.3 | 86.9 | 76.2 | 87.5 | 89.5 | 90.9 | 87.5 | 92.7 | 91.7 | 78 | 6.25 |
| PGT-126 | 149 | 133 | 89.5 | 79.2 | 84 | 85.7 | 91.7 | 78.9 | 83.6 | 78.1 | 87.3 | 91.7 | 78 | 5.71 |
| PGT-127 | 118 | 164 | 87.2 | 82.2 | 85.1 | 90.5 | 87.5 | 94.7 | 80 | 78.1 | 90.9 | 87.5 | 82.9 | 5.74 |
| PGT-128 | 173 | 109 | 64.2 | 92.5 | 81.6 | 81 | 66.7 | 94.7 | 83.6 | 87.5 | 89.1 | 70.8 | 70.7 | 10.11 |
| PGT-130 | 146 | 136 | 82.4 | 87.7 | 85.1 | 81 | 87.5 | 78.9 | 85.5 | 84.4 | 87.3 | 87.5 | 82.9 | 3.23 |
| PGT-131 | 101 | 181 | 90.6 | 76.2 | 85.5 | 85.7 | 79.2 | 78.9 | 89.1 | 96.9 | 80 | 87.5 | 80.5 | 6.33 |
| PGT-135 | 66 | 216 | 98.1 | 77.3 | 93.3 | 95.2 | 100 | 89.5 | 98.2 | 87.5 | 94.5 | 87.5 | 92.7 | 4.72 |
| PGT-136 | 31 | 251 | 99.2 | 77.4 | 96.8 | 100 | 100 | 94.7 | 98.2 | 90.6 | 98.2 | 100 | 92.7 | 3.67 |
| PGT-137 | 31 | 251 | 100 | 61.3 | 95.7 | 100 | 100 | 94.7 | 94.5 | 93.8 | 96.4 | 95.8 | 95.1 | 2.42 |
| | 121 | 161 | 92.5 | 90.1 | 91.5 | 66.7 | 95.8 | 84.2 | 94.5 | 93.8 | 98.2 | 83.3 | 92.7 | 10.35 |
| | 139 | 143 | 90.9 | 89.9 | 90.4 | 100 | 83.3 | 89.5 | 87.3 | 90.6 | 90.9 | 91.7 | 95.1 | 4.98 |
| | 140 | 142 | 89.4 | 92.9 | 91.1 | 95.2 | 79.2 | 94.7 | 92.7 | 84.4 | 87.3 | 95.8 | 100 | 6.91 |
| | 73 | 209 | 96.7 | 86.3 | 94 | 100 | 91.7 | 94.7 | 92.7 | 93.8 | 96.4 | 87.5 | 95.1 | 3.64 |
| | 132 | 150 | 89.3 | 91.7 | 90.4 | 85.7 | 87.5 | 84.2 | 90.9 | 93.8 | 90.9 | 91.7 | 95.1 | 3.84 |
| PGV04 | 216 | 66 | 72.7 | 95.4 | 90.1 | 85.7 | 95.8 | 84.2 | 94.5 | 90.6 | 89.1 | 95.8 | 82.9 | 5.22 |

EP 4 050 609 B1

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

## Patent documents cited in the description

- US 61637849
- US 61698614
- US 61751124
- US 61788529
- WO 2011119484 A
- US 4683202 A
- US 4683195 A
- US 4800159 A
- US 4965188 A

## Non-patent literature cited in the description

- **WALKER et al.** Broad and potent neutralizing antibodies from an African donor reveal a new HIV-1 vaccine target. *Science*, 2009, vol. 326 (5950), 285-89
- **FALKOWSKA et al.** PGV04, an HIV-1 gp120 CD4 binding site antibody, is broad and potent in neutralization but does not induce conformational changes characteristic of CD4. *Journal of Virology*, 2012, vol. 86 (8), 4394-4403
- **RUSSELL et al.** *Proc. Nat. Acad. Sci. USA*, 1979, vol. 76, 5918-5922
- **RUSSELL, W.** *Environmental Mutagens and Carcinogens: Proceedings of the Third International Conference on Environmental Mutagens*, 1982
- **SAMBROOK et al.** Molecular Cloning: A Laboratory Manual. Cold Spring Harbor Laboratory, 2001
- **AUSUBEL et al.** Current Protocols in Molecular Biology. Greene Publishing Associates and Wiley Interscience, 1989
- **KAN** ; **DOZY**. *Lancet*, 1978, vol. 11, 910-912
- **FAHAM** ; **COX**. *Genome Res.*, 1995, vol. 5, 474-482
- **WAGNER et al.** *Nucl. Acids Res.*, 1995, vol. 23, 3944-3948
- **FISHER et al.** *Proc. Natl. Acad. Sci. U.S.A.*, 1983, vol. 80, 1579-83
- **ORITA et al.** *Genomics*, 1983, vol. 5, 874-879
- **MYERS et al.** *Science*, 1985, vol. 230, 1242
- **COTTON et al.** *Proc. Natl. Acad. Sci. U.S.A.*, 1988, vol. 85, 4397-4401
- **YOUIL et al.** *Proc. Natl. Acad. Sci. U.S.A.*, 1995, vol. 92, 87-91
- **SYVANEN et al.** *Genomics*, 1990, vol. 8, 684-692
- **NIKIFOROV et al.** *Nucl. Acids Res.*, 1994, vol. 22, 4167-4175
- **LANDEGREN et al.** *Science*, 1988, vol. 241, 1077
- **BARRANY**. *Proc. Natl. Acad. Sci. U.S.A.*, 1991, vol. 88, 189-193
- **ABRAVAYA et al.** *Nucl. Acids Res.*, 1995, vol. 23, 675-682
- **ORUM et al.** *Nucl. Acids Res.*, 1993, vol. 21, 5332-5356
- **THIEDE et al.** *Nucl. Acids Res.*, 1996, vol. 24, 983-984
- **SOUTHERN**. *J. Mol. Biol.*, 1975, vol. 98, 503-517
- **ORITA et al.** *Proc. Natl. Acad. Sci. USA*, 1989, vol. 86, 2766-2770
- PCR Strategies. Academic Press, Inc., 1995
- **SANGER et al.** *Proc. Natl. Acad. Sci. USA*, 1977, vol. 74, 5463
- **MESSING et al.** *Nuc. Acids Res.*, 1981, vol. 9, 309
- **MAXAM et al.** *Methods in Enzymology*, 1980, vol. 65, 499
- **ZHANG et al.** Prediction of conformational B-cell epitopes from 3D structures by random forest with a distance-based feature. *BMC Bioinformatics*, 2011, vol. 12, 341
- **SINGH** ; **AHMAD**. *BMC Structural Biology*, 2009, vol. 9, 25
- **PINTAR et al.** Atom depth as a Descriptor of protein interior. *Biophysical Journal*, 2003, vol. 84, 2553-2561
- Remington's Pharmaceutical Sciences. Mack Publishing Company, 1980
- **RICHMAN et al.** *Proc. Natl. Acad. Sci.*, 2003, vol. 100 (7), 4144-49
- **SINGH et al.** Context dependent reference states of solvent accessibility derived from native protein structures and assessed by predictability analysis. *BMC Structural Biology*, 2009
- **DOORES** ; **BURTON**. Variable Loop Glycan Dependency of the Broad and Potent HIV-1-Neutralizing Antibodies PG9 and PG16. *Journal of Virology*, 2010, vol. 84, 10510-10521

• **WALKER et al.** Broad and potent neutralizing antibodies from an African donor reveal a new HIV-1 vaccine target.. *Science*, 2009, vol. 326 (5950), 285-89